(19)

Europäisches Patentamt

European Patent Office

Office européen des brevets

(11) **EP 3 231 478 A1**

(12) **EUROPEAN PATENT APPLICATION**

(43) Date of publication:
18.10.2017 Bulletin 2017/42

(21) Application number: **16165109.6**

(22) Date of filing: **13.04.2016**

(51) Int Cl.:
*A61N 1/40* (2006.01)      *A61N 5/10* (2006.01)
*A61K 41/00* (2006.01)      *B82Y 5/00* (2011.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Oncotherm Kft.**
**2040 Budaörs (HU)**

(72) Inventors:
• SZÁSZ, Olivér
2071 Páty (HU)
• ILURI, Nora
TX 75230 (US)
• SZÁSZ, Andras
2071 Páty (HU)

(74) Representative: **Arth, Hans-Lothar**
**ABK Patent Attorneys**
**Jasminweg 9**
**14052 Berlin (DE)**

(54) **RADIOFREQUENCY HYPERTHERMIA DEVICE WITH DOUBLE IMPEDANCE MATCHING SYSTEM**

(57)      The present invention relates to a radiofrequency (RF) hyperthermia device comprising a radiofrequency carrier signal generator **(1),** a radiofrequency amplifier **(4),** an electrode **(6)** and a counter-electrode **(7),** a first impedance measuring means **(2),** a first impedance matching means **(3),** a second impedance measuring means **(5),** and at least one second impedance matching means **(8** or **8'),** wherein the at least one second impedance matching means is configured and adapted to match the second impedance measured by the second impedance measuring means to the predetermined value. This radiofrequency (RF) hyperthermia device is designed for the hyperthermia treatment by optimized double impedance matching system.

Figure 10

EP 3 231 478 A1

## Description

## Specification

The present invention relates to a radiofrequency (RF) hyperthermia device comprising a radiofrequency carrier signal generator, a radiofrequency amplifier, an electrode and a counter-electrode, a first impedance measuring means, a first impedance matching means, a second impedance measuring means, and at least one second impedance matching means, wherein the at least one second impedance matching means is configured and adapted to match the second impedance measured by the second impedance measuring means to the predetermined value This radiofrequency (RF) hyperthermia device is designed for the hyperthermia treatment by optimized double impedance matching system.

## Description of the invention

The present invention is direct to a radiofrequency hyperthermia device comprising:

a radiofrequency carrier signal generator **(1)** configured and adapted to generate a radiofrequency carrier signal **(S1)** having a carrier frequency;

a radiofrequency amplifier **(4)** configured and adapted to amplify the radiofrequency carrier signal;

an electrode **(6)** and a counter-electrode **(7),** wherein a RF current is generated between the electrode **(6)** and the counter-electrode **(7)** for capacitive coupled energy transfer;

a first impedance measuring means **(2)** configured and adapted to measure a first impedance **(I1)** caused by the device;

a first impedance matching means **(3)** configured and adapted to match the first impedance **(11)** of the radiofrequency carrier signal **(S1)** measured by the first impedance measuring means **(2)** to a predetermined value;

a second impedance measuring means **(5)** configured and adapted to measure a second impedance **(12) ;** and

at least one second impedance matching means **(8 or 8')**;

characterized in that the at least one second impedance matching means **(8 or 8')** is configured and adapted to match the second impedance **(12)** measured by the second impedance measuring means **(5)** to the predetermined value.

In this embodiment, the second impedance is the second impedance of a radiofrequency carrier signal **(S3)** after amplification, which is caused by a target **(17)**:

In one embodiment of the present invention, the at least one second impedance matching means **(8 or 8')** of the inventive radiofrequency hyperthermia device is selected from the group consisting of: a tuneable coil, a capacitor and a coil in serial connection.

Preferred, the at least one second impedance matching means **(8 or 8')** of the inventive radiofrequency hyperthermia device is mounted on the electrode **(6)** and/or the counter-electrode **(7).**

In another embodiment, the at least one second impedance matching means **(8 or 8')** of the inventive radiofrequency hyperthermia device is integrated into the electrode **(6)** and/or the counter-electrode **(7).**

Preferred, the electrode **(6)** or the counter-electrode **(7)** of the inventive radiofrequency hyperthermia device is configured and adapted to any shape of a target **(17).**

In one embodiment, the target of the radiofrequency hyperthermia device is a tissue, an organ, a part or hole of a human or an animal body.

In one embodiment, the first impedance measuring means **(2)** and the first impedance matching means **(3)** of the radiofrequency hyperthermia device are integrated into the radiofrequency carrier signal generator **(1).**

In further embodiments, the radiofrequency hyperthermia device further comprises:

a modulation signal generator **(9)** configured and adapted to generate a modulation signal **(SM);**

a modulator **(11)** configured and adapted to convert the radiofrequency carrier signal **(S2)** obtained after the first impedance matching and the modulation signal **(SM)** generated by the modulation signal generator **(9)** to a modulated radiofrequency carrier signal **(S2M),**

wherein the radiofrequency amplifier (4) is configured and adapted to amplify the modulated radiofrequency carrier signal **(S2M)** converted by the modulator **(11).**

In these embodiments, the second impedance is the second impedance of a modulated radiofrequency carrier signal **(S3M)** after amplification, which is caused by the target **(17)**:

Preferred, the modulator of the inventive radiofrequency hyperthermia device is configured and adapted to modulate an amplitude of the radiofrequency carrier signal based on the modulation signal **(SM).**

More preferred, the modulator of the radiofrequency hyperthermia device is configured and adapted to further modulate a frequency of the radiofrequency carrier signal **(S2).**

In another embodiment, the radiofrequency hyperthermia device further comprises:

a modulation signal generator **(9)** configured and adapted to generate a modulation signal **(SM')**;

a modulator **(11)** configured and adapted to convert the radiofrequency carrier signal **(S2)** obtained after the first impedance matching and a modified modulation signal **(SM')** compensated by a feedback signal **(ST)** to a target modified modulated radiofrequency carrier signal **(S2M')**; and

a sensor **(12)** configured and adapted to detect a reflected or transmitted feedback signal **(ST)**;

wherein the modulation signal generator **(9)** receives a feedback signal **(ST)** detected by means of the sensor **(12)** for further generating the modified modulation signal **(SM')** compensated by the feedback signal.

Optionally, a signal checking unit **(15)** can be further configured to control the target modified modulated radiofrequency carrier signal **(S2M')**.

In addition, the feedback signal **(ST)** can be amplified to an amplified feedback signal **(ST2)** by means of a feedback signal amplifier **(14)** and in this case the modulation signal **(SM')** is compensated by the amplified feedback signal **(ST2)**.

In this embodiment, the second impedance is the second impedance of a target modified modulated radiofrequency carrier signal **(S3M')** after amplification, which is caused by the target **(17)**.

The present invention also includes a method for matching an impedance of a radiofrequency carrier signal of the radiofrequency hyperthermia device comprising the septs of:

(a) providing a radiofrequency carrier signal **(S1)** by means of a radiofrequency carrier generator **(1)**;

(b) measuring an impedance **(I1)** of the radiofrequency carrier signal **(S1)** by means of a first impedance measuring means **(2)**;

(c) matching the impedance **(I1)** of the radiofrequency carrier signal **(S1)** by means of a first impedance matching means **(3)** to obtain an radiofrequency carrier signal **(S2)** obtained after the first impedance matching;

(d) amplifying the radiofrequency carrier signal **(S2)** obtained after the first impedance matching by means of a radiofrequency amplifier **(4)** to obtain an amplified radiofrequency carrier signal **(S3)**;

(e) measuring an impedance **(I2)** directly at a target **(17)** by means of a second impedance measuring means **(5)**;

(f) compensating the impedance **(I2)** by means of at least one second impedance matching means **(8 or 8')**;

(g) directing the impedance matched carrier signal to an electrode **(6)** ; and

(h) generating a RF current between the electrode **(6)** and a counter-electrode (7) for capacitive coupled energy transfer.

In one embodiment, the step (d) of the above-mentioned method can be replaced with the following steps (d1) and (d2):

(d1a) modulating the radiofrequency carrier signal **(S2)** with a modulation signal **(SM)** provided by means of a modulating signal generator **(9)** to obtain a modulated radiofrequency carrier signal **(S2M)**;

(d2a) amplifying the modulated radiofrequency carrier signal **(S2M)** by means of a radiofrequency amplifier **(4)** to obtain a modulated radiofrequency carrier signal **(S3M)** after amplification.

Optionally, a further step (d1a') can be performed between the step (d1a) and (d2a) as follows:

(d1a') controlling the modulated radiofrequency carrier signal **(S2M)** by means of a signal checking unit **(15)**.

In another embodiment, the modulation signal can be compensated by a feedback signal **(ST),** and the steps (d1a) and (d2a) of the above-mentioned method can be replaced with the alternative steps (d1b) and (d2b):

(d1b) modulating the radiofrequency carrier signal **(S2)** with a modified modulation signal **(SM')** provided by means of a modulating signal generator **(9)** to obtain a target modified modulated radiofrequency carrier signal **(S2M')**;

(d2b) amplifying the target modified modulated radiofrequency carrier signal **(S2M')** by means of a radiofrequency amplifier **(4)** to obtain a target modified modulated radiofrequency carrier signal **(S3M')** after amplification.

Optionally, a further step (d1 b') can be performed between the step (d1 b) and (d2b) as follows:

(d1b') controlling the target modified modulated radiofrequency carrier signal **(S2M')** by means of a signal checking unit **(15)**;

In this embodiment, the method further comprises the following steps (i) and (j) after the step (h):

(i) detecting a feedback signal **(ST)** received by means of a sensor **(12);**
(j) sending the feedback signal **(ST)** received to the modulation signal generator **(9)** for providing a modified modulation signal **(SM')** compensated by the feedback signal in the step (d1 b).

Optionally, the feedback signal **(ST)** can be amplified and thus the method further comprises the following steps (i), (i') and (j) after the step (h):

(i) detecting a feedback signal **(ST)** received by means of a sensor **(12);**
(i') amplifying the feedback signal **(ST)** by means of an amplifier **(14)** to an amplified feedback signal **(ST2);**
(j') sending the amplified feedback signal **(ST2)** received from the amplifier **(14)** to the modulation signal generator (9) for providing a modified modulation signal **(SM')** compensated by the feedback signal in the step (d1 b).

Therefore, the steps (d1a), (d1a'), (d12a), (d1b), (d1b'), (d2b), (i), (i'), (j) and (j') can be replaced and/or combined with the steps (a)-(h) as follows:

- step (a) → step (b) → step (c) → step (d1a) → step (d2a) → step (e) → step (f) → step (g) → step (h);
- step (a) → step (b) → step (c) → step (d1a) → step (d1a') → step (d2a) → step (e) → step (f) → step (g) → step (h);
- step (a) → step (b) → step (c) → step (d1 b) → step (d2b) → step (e) → step (f) → step (g) → step (h) → step (i) → step (j);
- step (a) → step (b) → step (c) → step (d1b) → step (d1b') → step (d2b) → step (e) → step (f) → step (g) → step (h) → step (i) → step (j);
- step (a) → step (b) → step (c) → step (d1 b) → step (d2b) → step (e) → step (f) → step (g) → step (h) → step (i) → step (i') → step (j'); or
- step (a) → step (b) → step (c) → step (d1b) → step (d1b') → step (d2b) → step (e) → step (f) → step (g) → step (h) → step (i) → step (i') → step (j').

**Detailed Description of the invention**

**Impedance in the radiofrequency range**

The load on various electromagnetic sources absorbs the energy. However, its efficacy very much depends on the matching, how the load and the source are connected. The matching could target the elimination of the wave-reflection or the elimination of the imaginary part of the impedance. The previous is typically applied when the energy-transport is delivered basically by wave, and the last is when the quasi-static fields determine the matching. Waves are important when the distance of the source and target is more than the wavelengths of the given frequency (far-field approximation), while it is smaller than the impedance matching by compensating of the imaginary part of impedance is in practice (near-field approximation).

In therapeutic applications of radiofrequency in most of the cases near-field approach is valid, the electrodes are much nearer to the target than the wavelength of the applied frequency.

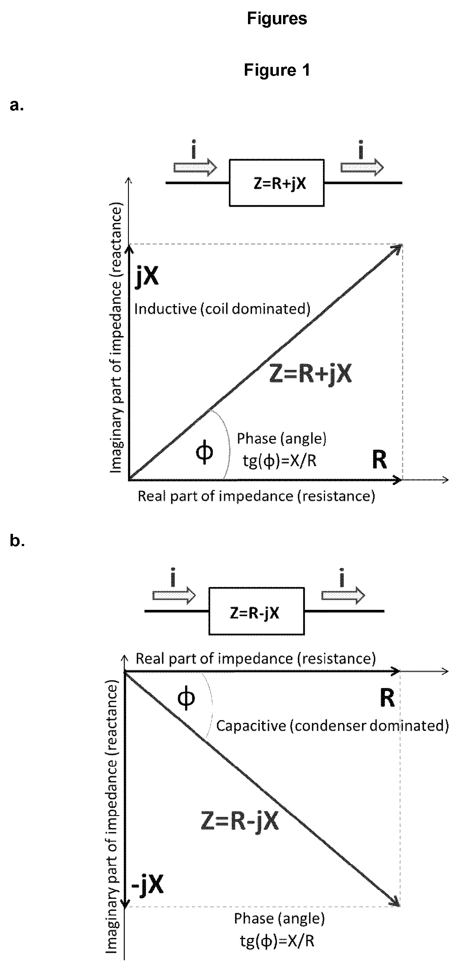

When the target is simply resistive (no capacitive or inductive component is involved), the current and voltage are in phase and the power could be calculated like it is in a simple direct current circuit, P=U*I, where P is the power, U is the voltage and I is the current. However, the problem starts, when capacitor or inductor (coil) appears in the system: the current and voltage will not be in phase and their phase angle ($\varphi$) determines the effective power in the target: P=U*I*cos($\varphi$). The impedance became a complex number additionally to the real one, the imaginary part ($j2=-1$) appear Fig.1.

In case of simple characterization of the inductive circuit by L induction at circular frequency w; we get:

$$X = j\omega L$$

While in case of capacitive by C capacitance:

$$X = \frac{1}{j\omega C} = -\frac{j}{\omega C}$$

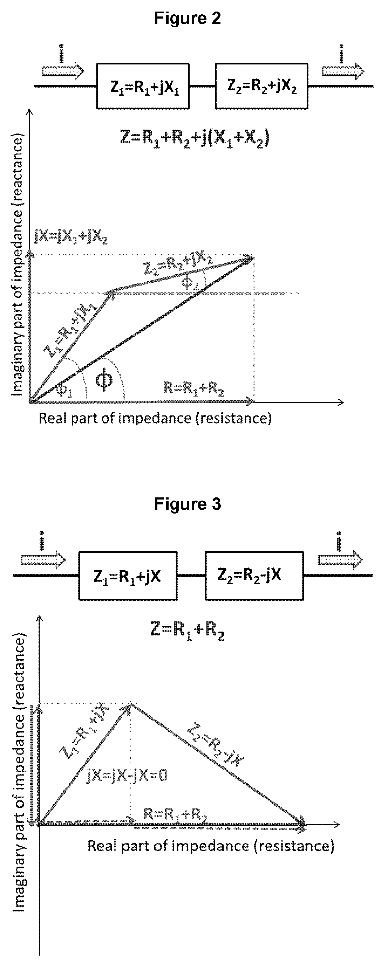

The complex numbers can be handled like vectors, so their serial connection could be described with the vectorial addition, Fig. 2.

When we add together an inductive and capacitive part with the same absolute value of imaginary part, then the system looks like a simple real-resistive one, Fig. 3.

In this case the inductive-X and capacitive-X eliminate each other, so

$$j\omega L - \frac{j}{\omega C} = 0$$

In consequence, it happens in a definite frequency only, which we call resonance frequency:

$$\omega_{resonance}^2 = \frac{1}{LC}$$

**Impedance of the living body**

The living body impedance is a subject of intensive research. Even numerous commercial products concentrate on its measurement bioimpedance analysis (BIA) calibrating the ideal electrolyte composition to the actually measured one. The electrical-impedance tomography (EIT) and electro-impedance spectroscopy (EIS) are accepted methods in medical diagnosis.

Multiple experimental and theoretical proofs are available proving the tight connection of the impedance changes to their structures. Impedance tomography as diagnostic method is also available to characterize the tissue by their impedance; even a routine determination of electrolytes is offered by the method.

The main function of the impedance matching compensates the reactant part of the impedance, and transforms it to the real resistive load. Consequently, the matching conditions, which fit to the impedance of the target, could follow the curative changes in the tissue level allowing the permanent control of the actual heating treatment.

Electric bio-impedance is a well-known method and a simple electrical-measurement technique. It measures the tissue's specific electric field distribution. The measurement uses the special frequency dispersion of the actual tissue. As early as in 1940 both the whole-body electrolyte status and the local changes (ECG) were studied by the method. Nowadays, it is widely applied, also for breast tumor diagnostics owning FDA approval (4/16/1999).

The bio-impedance is an investigated and applied diagnostic method to select the tissues on their impedance differences. Both the conductivity and dielectric differences between the healthy- and tumor-tissues at the applied 13.56 MHz frequency are over 15%. The impedance selectivity is applied in tomography device (Siemens oncological impedance-tomography device). The main challenge in the electro-impedance tomography (EIT) is the so-called "inverse problem": reestablishing the internal impedance arrangement by the externally measurable data.

In the present invention, the direct way can be applied. the device of the present invention, uses the impedance differences in the body without questioning where they are. The only point is that they have to be in the treated volume somewhere, and all the other job is done self-selectively (direct problem). The location and the local resolution of the method has no significance at the treatment, the determination of the lesion is made by other (conventional off situ, like ultrasound, CT or MRI) methods.

The self-selective factor (which according to the wide-ranging literature, ranges from 20% to 4000% showing the difference between the healthy and malignant tissues). The data are sporadically fluctuating, but all are alike in the opinion: the tumor always has lower impedance than its healthy counterpart does. This is exactly what is used in the oncothermia technique.

In a simple theoretical investigation an elliptical "tumor" is introduced into an otherwise homogeneous body. Making use of the level sets of the appropriate Green's function, it is able to identify (approximately of course) the changes in conductivity between the tumor and the surrounding region.

A precise diagnostics had been established by careful calculation of electric impedance of human thorax as well, and the 3-D electrical impedance tomography is intensively studied.

The increase of the current density in the tumor could be visualized by the RF-CDI (radiofrequency current density image), which is a definite, MRI-conducted measurement of the real processes.

Why does the bio-impedance differ between the malignant and healthy/benign tissues?

- The malignant cells are in frequent and permanent cellular-division. The energy-consumption for the intensive division is higher than the energy requirement for the healthy cells in homeostasis. The higher metabolic rate can be measured by positron emission tomography (PET). The higher metabolism increases the ion-transport and the ion-concentration in the area of the malignant cell, which lowers the impedance (gains the conductivity) of that volume. This effect is well-supported by the Warburg-theory, which has it renaissance in the research laboratories. Further effect is expected by the oncothermia: the intensive heat transfer on the cellular membrane intensifies the ionic-transports, which (in positive feedback) changes the ionic motility and conductivity.

- In addition, the gain of the **blood perfusion** by the increasing temperature (below 39°C) will lower the impedance (increase the conductance), which is an additional positive feedback selectivity at the beginning of the treatment. In advanced cases, the blood-perfusion is increased by the neo-angiogenesis. This extra perfusion (generally till T=39°C) lowers the impedance (increases the conductivity), which is again a selectivity factor.

- The growing temperature creates a **positive feedback** by its natural increasing effect on the conductivity. This increases the selection by the increasing temperature. The measured gain of the selectivity is 2% in °C. Which is in the range of 36→43 °C, that is, 14% increase.

- The impedance measurement is able to predict the fate of the cellular population treated by hyperthermia, and the knowledge of the **temperature and the applied time were not required,** these parameters were irrelevant for the proper control.

- The prompt **necrotic effect** trivially changes the impedance. During the capacitive coupling of oncothermia most of the RF-current flows in the extracellular electrolyte, the cells are electronically capsulated (shielded) by their membrane by more than one-million V/m field-strength. Oncothermia is devoted to damage the membrane of the malignant cells.

When the membrane is damaged, this (until the cytoplasma was excluded from the conduction) area becomes the part of the conductive process and the conductivity drastically changes. (Before this, the impedance grows because the cells swell and the extracellular volume shrinks). The cellular volume and the forming edema are also measurable *in situ.* The destructive phase of hyperthermia is well measurable, the necrotic and even the mitochondrial damage, as well as the cellular histolysis are observable,. The process of the observed characteristic cellular response is based on the cellular swelling, progressive membrane damage, cellular shrinkage and subsequent progressive histolysis. In the measurements, the histological changes and the cellular swelling, the progressive membrane damage, the cellular shrinking and the progressive histolysis can be followed by impedance. The induced coagulative necrosis in human xenografts is reflected. A special self-similar structure, the hierarchical circuit is also assumed, which well agrees with the fractal physiology approaches and the connected dynamism (noises).

- The malignant **tissue water content** is higher than that of its healthy counterpart's. The significantly larger permittivity and conductivity in tumor-tissue *in vitro* is explained on this basis.

- Furthermore, the proliferating cells control their cell-volume by their water content, in the malignant growth, and this effect increases the conductivity in the given tissue, too.

- The malignancy has a special **fractal structure,** which can be identified by impedance measurements on Erlich solid tumors. This structure (due to its definite percolative self-similarity) is a better conductor than the non-fractal healthy tissue.

- The block of **cell-junctions** and the characteristic isolation of the malignant cells from each other. With this mechanism the extracellular pathway becomes free between the cells, definitely increasing the conductivity of the extracellular electrolyte.

- Furthermore, decrease of epithelial barrier function (tight-junction permeability changes) has a role in the development of colon tumors, which can be measured by electric impedance measurement.

- It is **temperature dependent** and hyperthermia can be monitored. Arrhenius activation energy is also measured by impedance.

- Numerous important **physiologic parameters,** like apoptosis, and ischemia can be followed also by monitoring the bio-impedance.

- The special **membrane effects** (rectification on membranes), are also a factor of the cellular selectivity of cancer by RF electric field.

- The impedance measurement is useful for the **control of other treatment** modalities. It adequately measures the distortion made by irradiation, and the drug-effect can also be controlled. Such usual practice, like following the wound healing, is also objectively traceable. Bioimpedance vector pattern can distinguish between cancer patients without disease versus locally advanced or disseminated disease.

- The method could be used for the **control of the tissue cultures;** causing significant impedance change by thickness and homogeneity of the culture-growth. Combining optical and electrical impedance techniques for quantitative measurement of confluence MDCK-I cell cultures. An interesting tissue and cell-culture characterization method has been established, (ECIS Method, masterminded and chaired by a Nobel-laureate; and it is widely used by the pharma-industry. In addition, the electric impedance could be sensitive for the drug-reactions. The dielectric loss is changed by chemo, which may also be a helping factor *in vivo,* when the chemo-infiltrated tumor has more ions, so less conductivity increasing the selectivity by oncothermia.

- In some hyperthermia cases, the impedance measurement becomes the **control of the treatment quality.** It is widely applied for RF ablation/interstitial technique without any control of the temperature.

The magnetic resonance electro-impedance tomography (MREIT) shows spectacular data on the impedance selectivity, to prove the theoretical calculations and selectivity assumptions.

The impedance measures selectively, differentiates between the cancerous and healthy tissue, and is able to distinguish the extra- and intra-cellular electrolyte. Selective impedance measurements are provided clinically as well. Many comparative studies were provided for malignant tissues, however, the results are not identical; the measurements very much depend on the conditions. This is a trivial consequence of many factors as listed above. However, all the studies measured lower impedance in the tumor than in their healthy counterpart, in all of the tissue and staging of the tumor, *in vitro* and *in vivo,* as well.

## Bioimpedance in practice

It is clinically proven, that the cancerous and healthy tissues of the **hepatic tumors** are significantly different. Also the VX-2 carcinoma can be measured: rabbit-liver at low frequency *in vivo* had a conductivity 6-7.5 times higher, permittivity 2-5 times lower than in healthy parts (impedance difference is about 600%), while for 10 MHz region it is 200%. By impedance tomography can be distinguished between the living and necrotic malignancy as well; both the conductivity and permittivity are higher in malignant liver, but the frequency dependence of necrotic tissue differs.

The impedance method is well applicable for **breast cancer** biopsies and for breast cancer diagnostics and prophylactics. The largest selectivity was measured in breast; (up to 40-times lower in the breast tumor than in the healthy environment). There are commercially available devices for the impedance tomography for mammography use. One of the best selections could be reached in the breast. The breast tissue is widely investigated and reviewed.

Also impedance tomography used for mammography purposes became a commercial tool to make breast-control with cheap and harmless procedure.

Clinically **advanced lung cancer** can also be followed by impedance observations It has been successfully tried in **skin-cancer** diagnosis. The significant separation of the malignant and benign tumors was successfully applied on skin. Temporal resolution of the skin-impedance measurement in frequency-domain method extended this application. Nodular **basal cell carcinoma** was checked, patients have significant impedance change measured in their tumor.

The smallest impedance difference was measured for **kidney tumors** (4-6%), but other measurements had basically different results for renal tumors: $1.43 \pm 0.39$ S/m, (while $0.73 \pm 0.77$ S/m for renal healthy (p<0.05) [96% increase].

In **colorectal cancer** the phase-angle investigations of electric impedance are good indicators of cellular health and integrity. In this manner, the phase angle is a prognostic indicator: low phase angle (below 5.17 10.1 months, 5.17-6.19 22.6 months, above 6.19 was 25.6 months). These data were statistically significant (p=0.003), independent from the tumor stage. Very promising detection possibility was shown for colon by preclinical investigations; where the conductance of the surface colonic epithelium conductance was significantly increased in cancer-susceptible distal colon (from $41.1 \pm 3$ mS/cm2 to $52.6 \pm 3.1$ mS/cm2 [28%]).

Electrical-impedance scanning, applied for lymph-node evaluation in **lymph-node evaluation** of children, is also successfully introduced.

This method, like a non-invasive biopsy control, was successfully applied for **Barrett's esophagus.**

The pre-malignant check by **non-invasive (virtual) biopsy,** is an option by the electro-impedance method, reducing the need for invasive biopsies.

The impedance differences can be used in **labor diagnosis:** the impedance spectroscopy is effective in early tumor-stages and single-cell characteristics, too. The method is cell-selective. The ECIS method measures the metastatic potential investigated in many leading laboratories in the USA.

The method can be applied in the **clinical prognosis.** The bioimpedance in whole body was successfully applied to distinguish between the patients without disease and the locally advanced or disseminated disease.

The **cell motility** probed by noise analysis of thickness shear mode resonators. The noise analyses of the electric currents became a new tool in the field.

Absorption and fluctuations of **giant liposomes** were studied by electrochemical impedance measurements.

## Impedance of malignant region

As described above, there are numerous robust differences between the impedance of malignant and healthy cells, which we may use for their recognizing. Selection of the malignant cells are based on four complex and interacting effects:

1. Differences of metabolic rate of the malignant and healthy cells (Warburg effect), leading to higher conductivity of tumor than its neighbors;

2. Higher dielectric constant of the extracellular electrolyte in immediate vicinity of the malignant cells compared to their healthy counterpart, (Szentgyorgyi effect);

3. The well-chosen frequency could select different parts of the anyway inhomogeneous tissue. These are called dispersion relations.

4. Structural differences (pathological pattern recognizing) of the malignant and healthy tissues (fractal physiology effect). The structural differences are again mirrored in the impedance of the tissues.

Use of these biophysical differences described in details makes the accurate selection of the cancer-cells possible and actively destroys them without damages on their healthy neighborhood. Here the dose is crucial: applying too much energy realizes the classical hyperthermia, it heats up all the components of the target, and we lose the selection and its consequent healing effects. Multiple hyperthermia devices deliver the heating energy by electromagnetic ways. The common technical solution of these is the matching of the load to the electromagnetic source by various methods. This matching optimizes and makes the electromagnetic coupling to the target effective by fitting of the impedance of the energy-source to the load impedance of the target. Permanent control of the matching governs the fitting process, and makes the optimal energy delivery possible despite the dynamically changing impedance of the target. The dynamic change in case of biological targets could be originated from the reorganization of the tissue-structure, the change of the electrolytes in biomatter, or caused by simple mechanical move during the treatment.

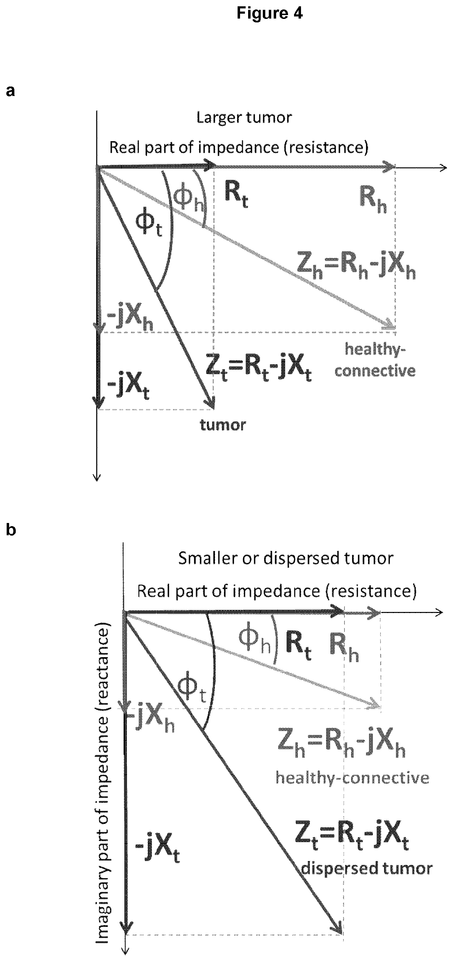

Due to the better conductivity and higher dielectric permittivity, the tumor impedance vector has usually larger decline from the horizontal axis than the healthy tissue in the same volume (Fig. 4). However, when the tumor is dispersed the angles can change considerably, mainly the conductivity factor in the tumor will not be as dominant as it was, while in the healthy part it would be more conductive.

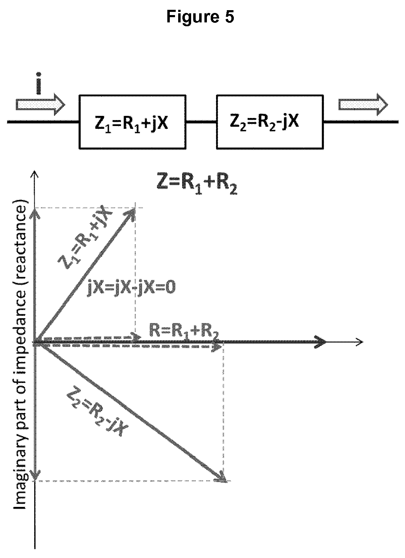

The decline of the impedance vector could be compensated by another vector (with opposite sign of the phase angle). The main criterial of the compensation are the identical absolute values but opposite signs of the reactance in the two vectors (Fig. 5).

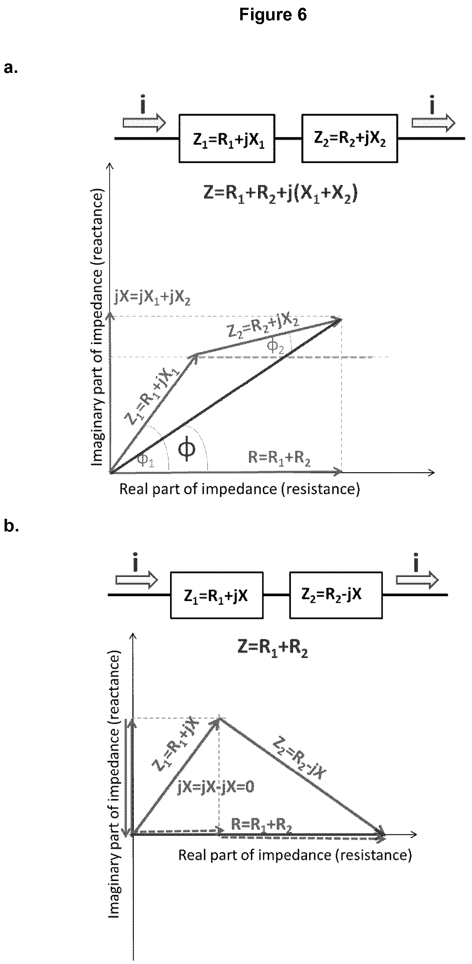

For easy vector-diagrams we put the various impedance vectors subsequently in a line, and the resulting impedance will be the linear connection at the two ends (Fig. 6).

**The impedance sequences of the body**

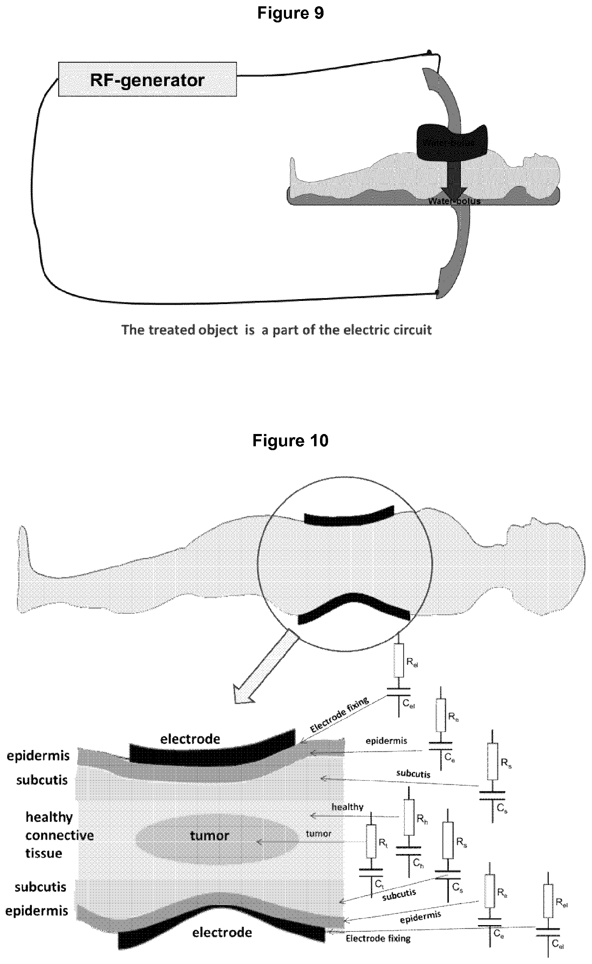

The capacitive impedance coupling is one of the most popular energy-delivery into the body. It is used frequently in oncology (hyperthermia) to eliminate the tumor which is targeted by the electromagnetic energy. One kind of this method is oncothermia (Fig. 9).

The living objects have only two types of the impedance elements: resistivity and capacitive reactance, no inductive element in macro level could be recognized. The capacitance is mainly originated from the membranes and walls which separate the various aqueous electrolytes in the body.

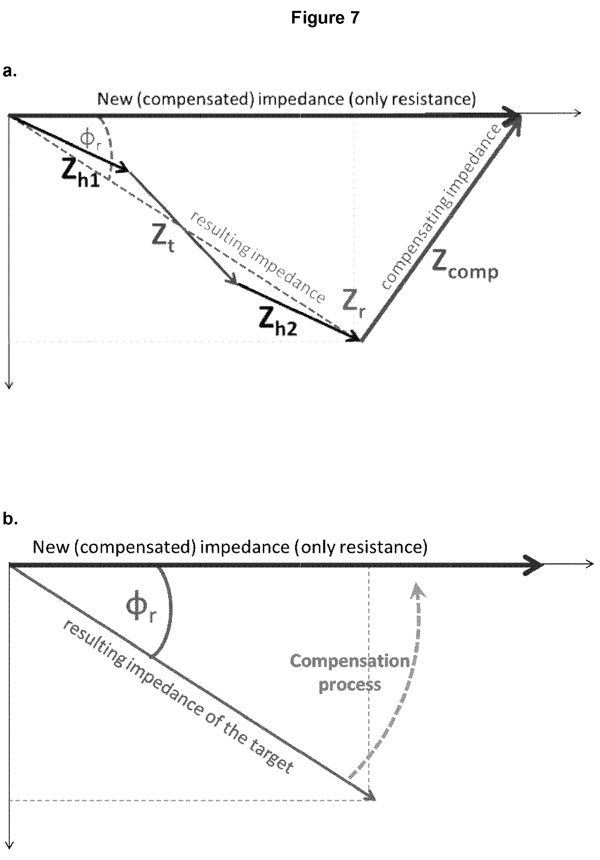

The impedance of the various tissues is summarized when the radiofrequency current flows through the complete cross section. Many impedance elements are complexly networked inside the body and also the impedance of the electrodes themselves modifies the resulting impedance. In the vicinity of the tumor the healthy environment could be counted (Fig. 9). The compensation of the imaginary part could have various types of actions (Fig. 7).

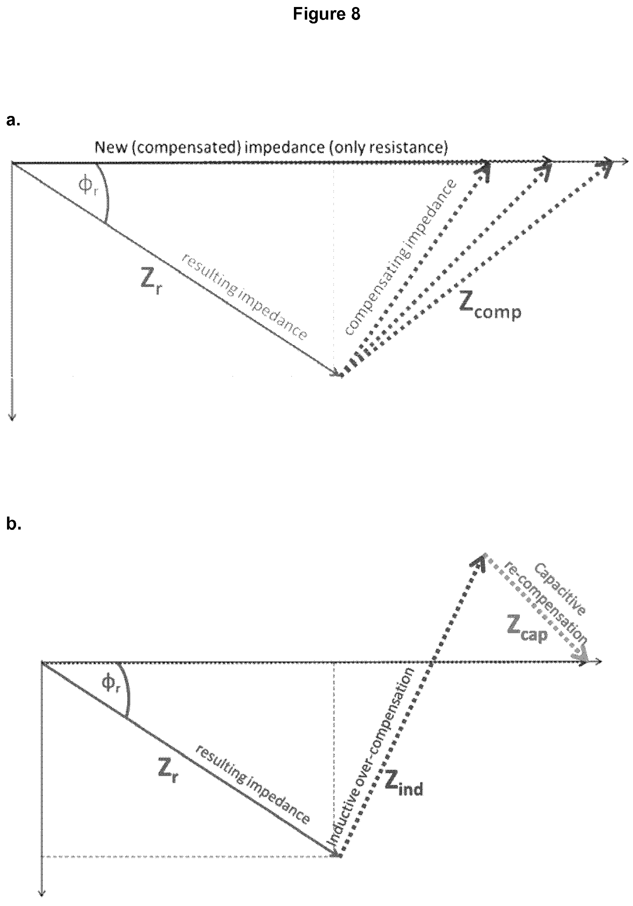

Of course, numerous heterogeneities are in the body between the electrodes, which impedances have to be added. For simplicity, it is shown here only some characteristic impedances: the epidermis, subcutis, healthy connective tissue and the tumor-tissue (Fig. 8). The electrodes (how they are fixed to the body) could be definite determination of the resulting impedance.

The resultant impedance could be calculated by vectorial additions in subsequent layers (Fig. 7).

The electrodes can be fixed on the way (varying its impedance) when the parts of the upper and lower level of the cross section are approximately symmetric. This is mainly electrode dependent, but it also depends on the dominance of the tumor impedance (Fig. 8).

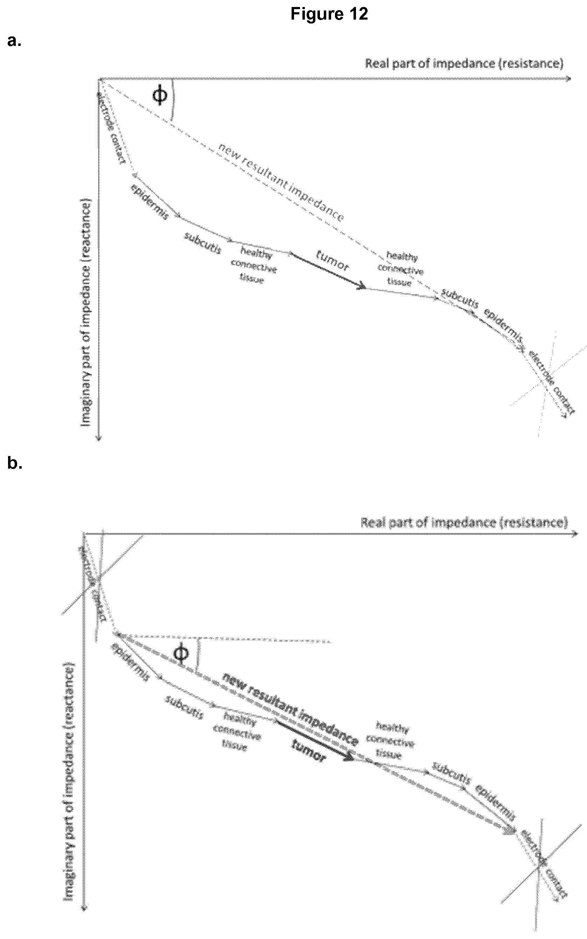

For the best efficacy in the tumor-energy-absorption, the impedance of the tumor has to be compensated to the solely real resistance, which is not able, when the electrodes are not directly connected to the tumor (invasive solution). When no such case is available, it can be tried to compensate the resultant impedance by the nearest compensation to tumor (zero phase-angle in the tumor). When the electrode impedance in one side is so strict that the imaginary part is small and neglected (for example a large grounded electrode or gelling or other boosting of the coupling), probably we can approach the zero phase-angle better in the tumor region. The resultant impedance could be compensated by the inductor (coil) when the resonant frequency is the definite applied RF (Fig. 12).

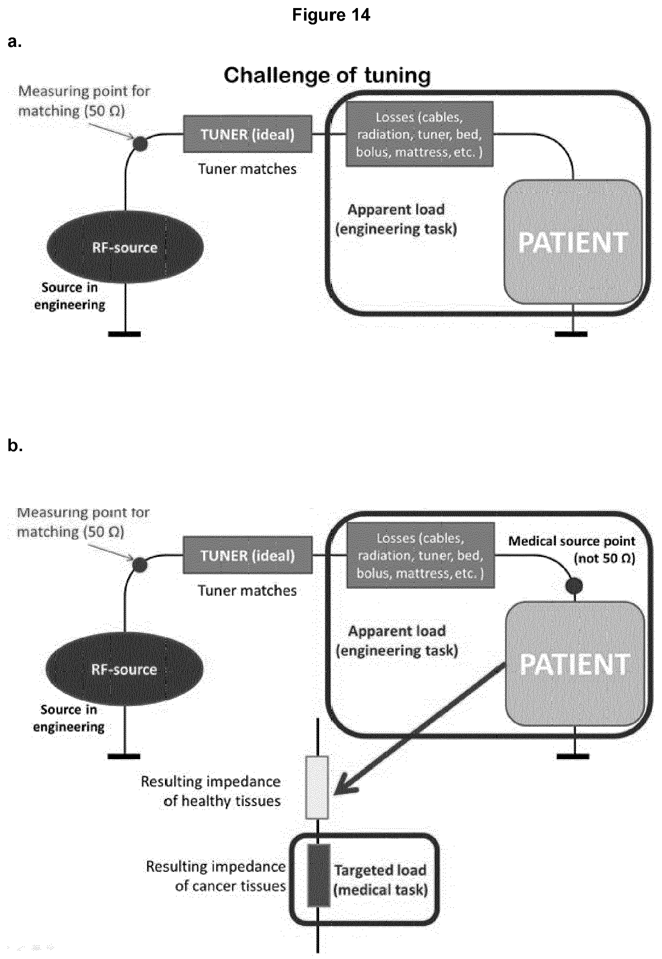

When the compensation is correct, the energy-absorption efficacy is the highest in the tumor. Do not mix this matching problem with the complete matching of the system (load electrodes, wires, radiative losses, etc.) to the power-source (amplifier) which is optimal when the system is compensated to the conventionally determined real 50 Ohm. This tuning is secondary, it could happen after the compensation of the imaginary part in the immediate vicinity of the treated target. Figures 14 provides a simplified concept of the inventive device.

The radiofrequency hyperthermia device of the prior art fails to compensate mismatched impedance caused by the target.

The object of the present invention is to provide a radiofrequency hyperthermia device for effective hyperthermia treatment, especially of cancer, and proliferative diseases by double impedance matching system.

This object is solved by the present invention, especially a radiofrequency hyperthermia device having double impedance matching system. This double impedance matching system increases the energy-absorption efficacy on the tumor and enables more selective and effective treatment of the tumor. Further advantageous features and embodiments are evident from the description, the examples and the dependent claims.

Thus, the present invention is direct to a radiofrequency hyperthermia device comprising:

> a radiofrequency carrier signal generator **(1)** configured and adapted to generate a radiofrequency carrier signal **(S1)** having a carrier frequency;

> a radiofrequency amplifier **(4)** configured and adapted to amplify the radiofrequency carrier signal;

> an electrode **(6)** and a counter-electrode **(7),** wherein a RF current is generated between the electrode **(6)** and the counter-electrode **(7)** for capacitive coupled energy transfer;

a first impedance measuring means **(2)** configured and adapted to measure a first impedance **(I1)** caused by the device;

a first impedance matching means **(3)** configured and adapted to match the first impedance **(I1)** of the radiofrequency carrier signal **(S1)** measured by the first impedance measuring means **(2)** to a predetermined value;

a second impedance measuring means **(5)** configured and adapted to measure a second impedance **(I2)** ; and

at least one second impedance matching means **(8 or 8')**;

characterized in that the at least one second impedance matching means **(8 or 8')** is configured and adapted to match the second impedance **(I2)** measured by the second impedance measuring means **(5)** to the predetermined value.

In general, the second impedance of the carrier signal is caused by a target **(17).** In this embodiment, the second impedance is the second impedance of a radiofrequency carrier signal **(S3)** after amplification, which is caused by a target **(17).** Therefore, alternatively, a radiofrequency hyperthermia device can be defined as follows:

A radiofrequency hyperthermia device of the present invention comprising:

a radiofrequency carrier signal generator **(1)** configured and adapted to generate a radiofrequency carrier signal **(S1)** having a carrier frequency;

a radiofrequency amplifier **(4)** configured and adapted to amplify the radiofrequency carrier signal;

an electrode **(6)** and a counter-electrode **(7),** wherein a RF current is generated between the electrode **(6)** and the counter-electrode **(7)** for capacitive coupled energy transfer through a target **(17);**

a first impedance measuring means **(2)** configured and adapted to measure a first impedance **(I1)** caused by the device;

a first impedance matching means **(3)** configured and adapted to match the first impedance **(I1)** of the radiofrequency carrier signal **(S1)** measured by the first impedance measuring means **(2)** to a predetermined value;

a second impedance measuring means **(5)** configured and adapted to measure a second impedance **(I2)** caused by the target **(17)** ; and

at least one second impedance matching means **(8 or 8')**;

characterized in that the at least one second impedance matching means **(8 or 8')** is configured and adapted to match the second impedance **(I2)** caused by the target (17) measured by the second impedance measuring means **(5)** to the predetermined value.

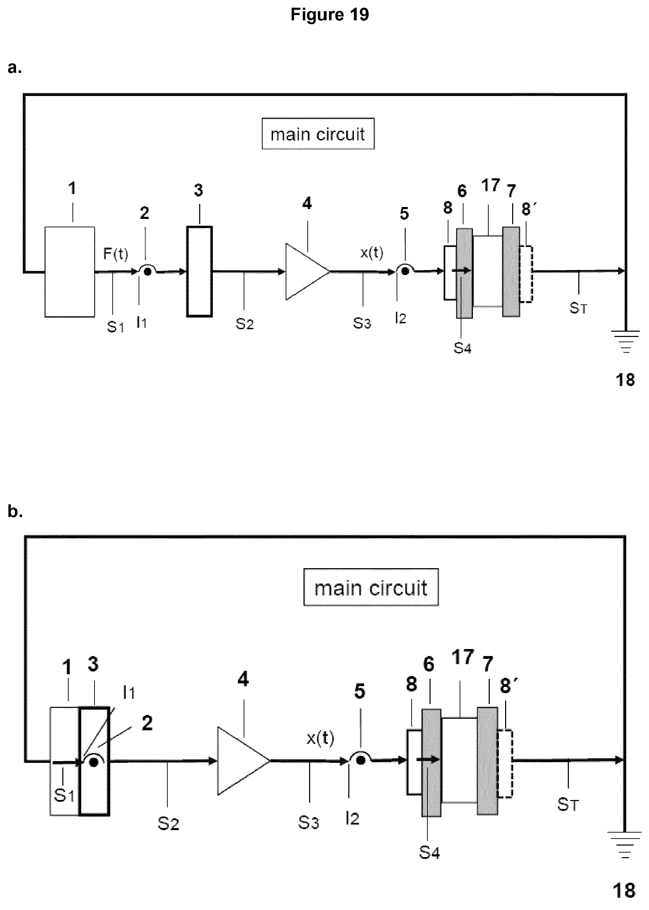

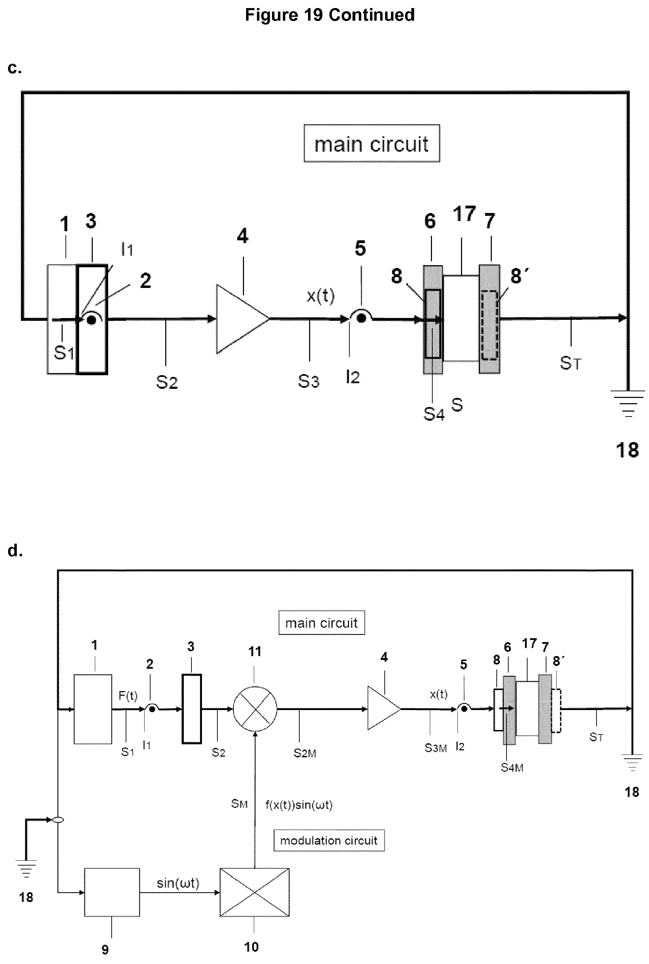

The above-described inventive device with the essential components is depicted in Figures 19 (a), (b) and (c).

**The radiofrequency carrier signal generator** generates a radiofrequency carrier signal. Preferably the RF signal is a free industrial frequency like 13.56 MHz or the double or the triple of 13.56 MHz.

**The first Impedance matching means**

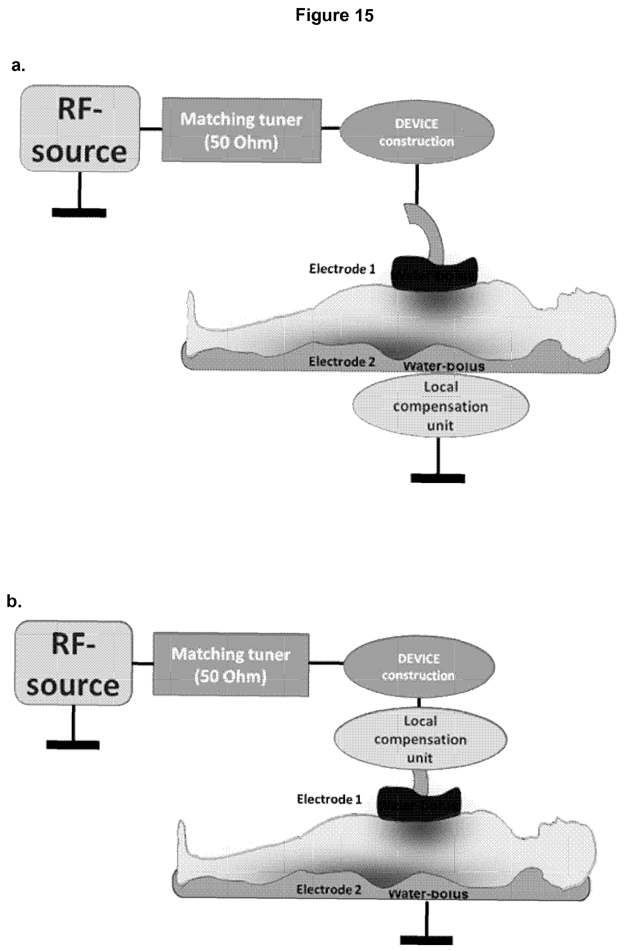

For optimal matching the impedance, the inventive device comprises the first impedance matching means. In the inventive device, the automatic impedance tuning system is applied as the first impedance matching means (Fig. 14 and 15). This first impedance matching means is configured to tune the applied RF wave (carrier signal) to the predetermined impedance value, preferably 50 Ohm.

The first impedance matching means can be controlled by a first impedance matching control system. The first impedance matching control system comprises at least calculation unit, tuning parameter data acquisition unit, command transmission unit and controller.

Preferred, the first impedance measuring means **(2)** is disposed upstream of the radiofrequency carrier signal generator **(1)** and the first impedance matching means **(3)** is disposed upstream of the first impedance measuring means **(2),** i.e. between the radiofrequency carrier signal generator **(1)** and the radiofrequency amplifier **(4).**

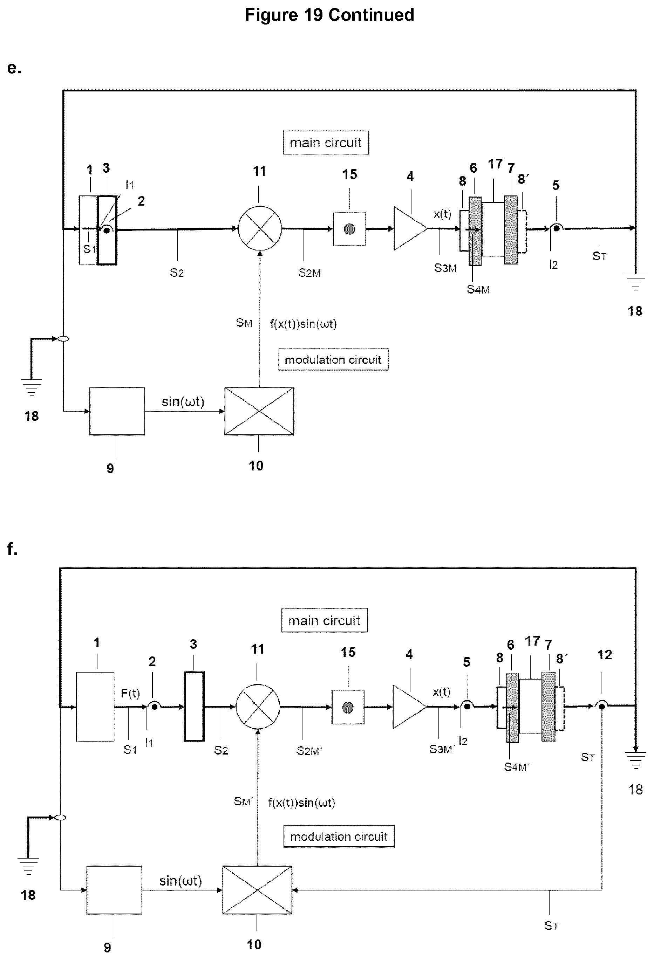

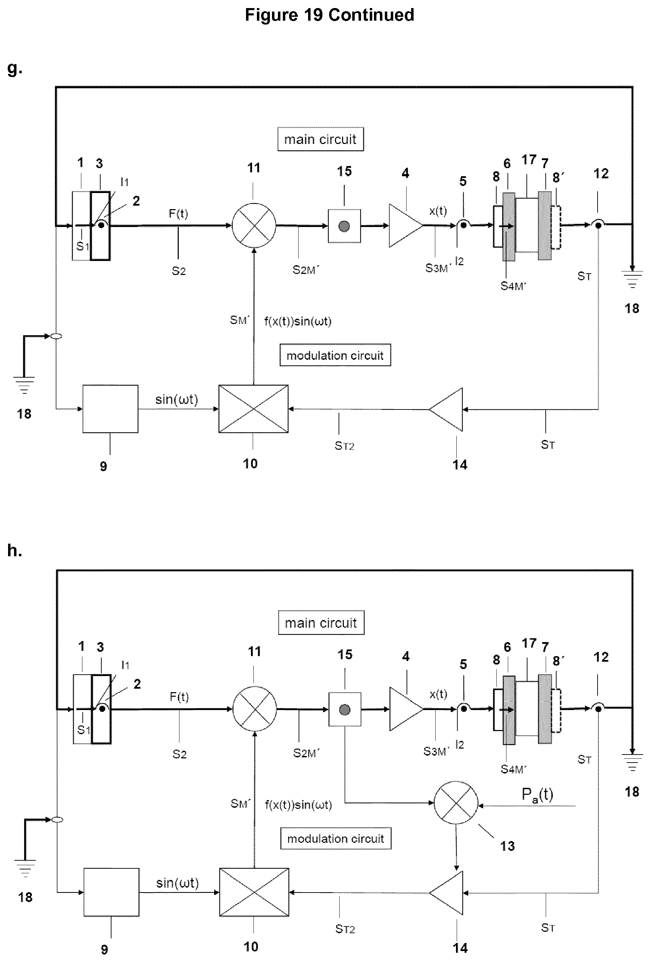

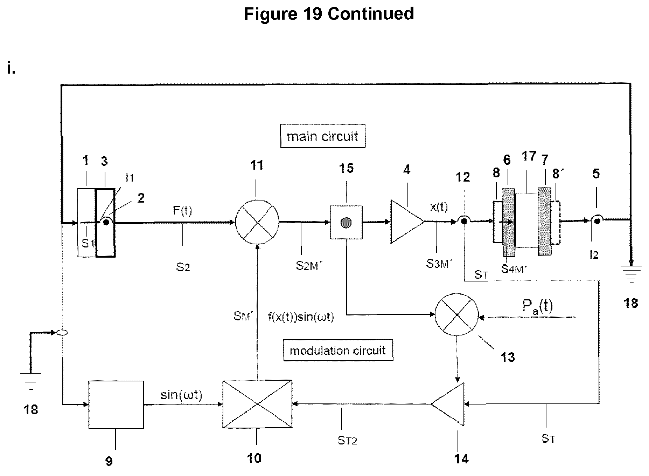

In one embodiment, the first impedance measuring means **(2)** and the first impedance matching means **(3)** of the radiofrequency hyperthermia device are integrated into the radiofrequency carrier signal generator **(1)** (Fig 19. b, c, e, g, h, and i).

**The second impedance matching means**

In the present invention, an impedance-matching directly is made at the target (the patient) and not in the device. This is different from the auto-matching in the device is untouched governing the optimal work of the RF-source and compensate the RF-losses in the circuits. The second impedance matching means is responsible for this task.

1. The second impedance matching (extra-matching) by means of the second impedance matching means makes the compensation where the reflection is minimal so it is as near as possible to the zero-phase-angle position. This position is measured by S11 parameter, which is a standard measurement of such kind of reflection.

2. The goal of the present invention is to have a near-zero phase angle in the tumor also, but we are not able to go into the body invasively to tune the tumor as an independent load, so the inventive device tunes the resulting complete impedance of the patient (but excluding all the circuit components of the RF-source and cabling, etc.).

3. The optimization of the tumor targeting could be done only when the impedance vector of the tumor and the resulting impedance of the patient is close to parallel, because then the compensation of the patient impedance makes the same zero-phase compensation for the tumor.

The proper matching can be measured with the S11 parameter, which measures the reflected power by the un-matched imaginary part of the impedance. The ideal matching, when the capacitive part of the impedance (only that exists in biosystems) is "compensated" by a proper induction. The arrangement makes resonant frequency on the carrier (frequently 13.56 MHz is used as medical standard, but could be any). The reciprocal value of the square of the resonant frequency is equal of the product of C capacity (to compensate) and L induction (actually making the match).

The second impedance matching unit (or an additional matching unit), which could change the L induction (this is the main matching). Furthermore, the capacity of the electrode arrangement (by pressure on the patient, by size of touching area, by position of the electrodes, etc.) may be adjusted, and offer the position which the range of L could manage.

Measurement components could be the S11 or (little more complicated) the phase angle between the voltage and current which flows through the patient. The phase angle must be minimized. The components could be only the actual C and L values at the electrode and patient complex system. The L value could be changed by a coil (as described in details in the application). The L-value has only one component, the coil which is applied in the present invention. The C-value has multiple components. It could depend on the target (patient) size, treated part of the body, tumor size, tumor-position, thickness of adipose tissue, pressure of the electrode to patient skin, size of the active area of the electrode, perspiration of the patient under the electrode, movement of the target (patient), etc. The product of LC has to be kept in optimal case on the resonance frequency. It could be automatic (feedback form S11 or phase-angle measurements) or could be manual indicating the request of action for the practitioner like doctor.

The optimal matching (resonant frequency) does not depend on the value of the absorbed energy. It is definite by $\omega_0 = \frac{1}{\sqrt{LC}}$ where $\omega_0$ is the resonance frequency, L is the actual (serial) induction value and C is the actual (serial) capacitance.

When the absorbed energy is large it could be only apparent, when the phase angle is also large. The goal of the second impedance matching is primarily the well matched position and makes the power in this condition. The power has a wide interval for action, but the reactive current is really contra-effective. So our primary goal is the matching, and uses with this the power in the effective interval. The effective interval of the power depends on the disease, and summarized in the protocols of the treatments.

The compensation is a coil or coil-condenser serial circuit in the complete treatment circuit, (Fig. 15). It does not need any change on the normal tuning (auto-matching) of the system for the best efficacy of the RF-source.

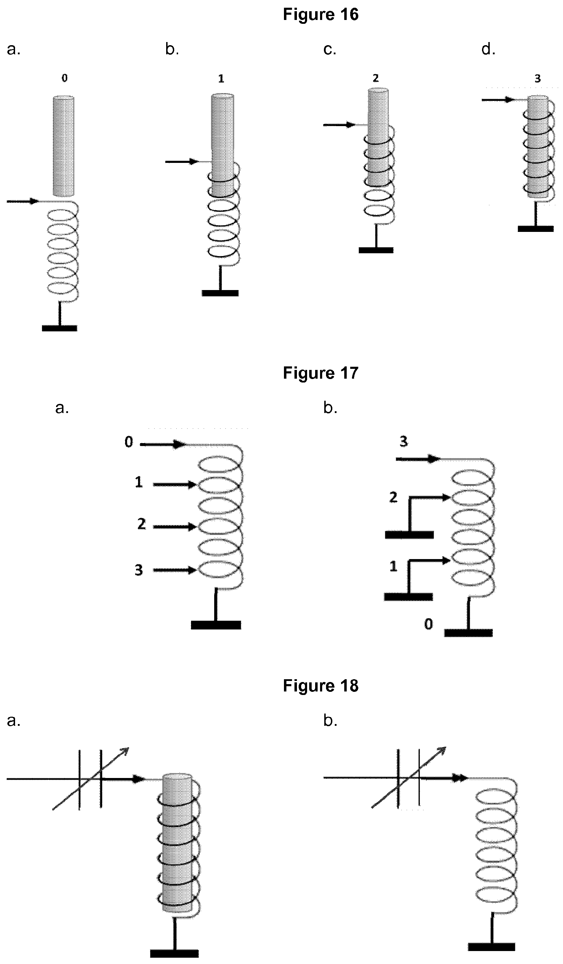

The coil compensation could be by varying the impedance of the coil by immersing material into a wire-coil (Fig. 16). Depending on the magnetic permeability of the material the range and the fine control could be adjusted.

A possible solution is having wired or core included coil with variation of their length and so by this variation of their inductivity (Fig. 17). This solution changes the galvanic resistivity connections in the circuit, while the other (preferable solution with core material) does not influence the normal resistivity of the coil.

Sometimes the control and tuning by varying capacitance is easier. In this case the over-tuned inductor could be re-compensated by this tunable capacitor, fixing the desired values on the given frequency (Fig. 18).

Therefore, in one embodiment of the present invention, the at least one second impedance matching means (8 or 8') of the inventive radiofrequency hyperthermia device is selected from the group consisting of: a tuneable coil, a capacitor and a coil in serial connection.

Preferred, the at least one second impedance matching means (8 or 8') of the inventive radiofrequency hyperthermia device is mounted on the electrode (6) or the counter-electrode (7) (Fig. 19a and 19b).

In another embodiment, the second impedance matching means (8 or 8') of the inventive radiofrequency hyperthermia device is integrated into the electrode (6) or the counter-electrode (7) (Fig. 19c).

## Modulator

In further embodiments as shown in Figures 19d and 19e, the radiofrequency hyperthermia device further comprises:

a modulation signal generator (9) configured and adapted to generate a modulation signal (SM);

a modulator **(11)** configured and adapted to convert the radiofrequency carrier signal **(S2)** after the first impedance matching and the modulation signal **(SM)** generated by the modulation signal generator **(9)** to a modulated radiofrequency carrier signal **(S2M),**

wherein the radiofrequency amplifier **(4)** is configured and adapted to amplify the modulated radiofrequency carrier signal **(S2M)** converted by the modulator **(11).**

Preferred, the modulator **(11)** of the inventive radiofrequency hyperthermia device is configured and adapted to modulate an amplitude of the radiofrequency carrier signal based on the modulation signal.

More preferred, the modulator of the radiofrequency hyperthermia device is configured and adapted to further modulate a frequency of the radiofrequency carrier signal.

In case the modulator **(11)** is present, the present invention relates to a radiofrequency hyperthermia device comprising:

a radiofrequency carrier signal generator **(1)** configured and adapted to generate a radiofrequency carrier signal **(S1)** having a carrier frequency;

a modulation signal generator **(9)** configured and adapted to generate a modulation signal **(SM);**

a modulator **(11)** configured and adapted to convert the radiofrequency carrier signal **(S2)** after the first impedance matching and the modulation signal **(SM)** generated by the modulation signal generator **(9)** to a modulated radiofrequency carrier signal **(S2M);**

a radiofrequency amplifier **(4)** configured and adapted to amplify the modulated radiofrequency carrier signal **(S2M)** converted by the modulator **(11);**

an electrode **(6)** and a counter-electrode **(7),** wherein a RF current is generated between the electrode **(6)** and the counter-electrode **(7)** for capacitive coupled energy transfer;

a first impedance measuring means **(2)** configured and adapted to measure a first impedance **(I1)** caused by the device;

a first impedance matching means **(3)** configured and adapted to match the first impedance **(I1)** of the radiofrequency carrier signal **(S1)** measured by the first impedance measuring means **(2)** to a predetermined value;

a second impedance measuring means **(5)** configured and adapted to measure a second impedance **(I2)** ; and

at least one second impedance matching means **(8 or 8');**

wherein the at least one second impedance matching means **(8 or 8')** is configured and adapted to match the second impedance **(I2)** measured by the second impedance measuring means **(5)** to the predetermined value.

In this embodiment, the second impedance is the second impedance of a modulated radiofrequency carrier signal **(S3M)** after amplification, which is caused by the target **(17).**

The modulation signal source is preferably a pink-noise (1/f noise) frequency generator. Pink noise has a fractal (time-domain) fluctuation, having long-range efficacy to act on the dynamical processes in a given target tissue/cell. It is preferably used for amplitude modulation. Pink noise modulation is preferable, but the pink noise spectrum is not necessary in all cases.

The modulation signal modulates the carrier signal to create modulated radiofrequency carrier signal **(S2M)** by amplitude modulation, frequency modulation or phase modulation. Preferably the signal is modulated by amplitude modulation, as outlined above.

In another embodiment as shown in Figure 19f, the radiofrequency hyperthermia device further comprises:

a modulation signal generator **(9)** configured and adapted to generate a modulation signal **(SM),** wherein the modulation signal generator **(9)** further receives a feedback signal **(ST)** detected by means of the sensor **(12)** for further generating the modified modulation signal **(SM')** compensated by the feedback signal **(ST);**

a sensor **(12)** configured and adapted to detect a reflected or transmitted feedback signal **(ST);** and

a modulator **(11)** configured and adapted to convert the radiofrequency carrier signal **(S2)** obtained after the first impedance matching and a modified modulation signal **(SM')** compensated by a feedback signal **(ST)** to a target modified modulated radiofrequency carrier signal **(S2M').**

In case the feedback signal is present, therefore the present invention relates to a radiofrequency hyperthermia device comprising:

a radiofrequency carrier signal generator **(1)** configured and adapted to generate a radiofrequency carrier signal **(S1)** having a carrier frequency;

a modulation signal generator **(9)** configured and adapted to generate a modulation signal **(SM),** wherein the modulation signal generator **(9)** further receives a feedback signal **(ST)** detected by means of the sensor **(12)** for further generating the modified modulation signal **(SM')** compensated by the feedback signal **(ST);**

a modulator **(11)** configured and adapted to convert the radiofrequency carrier signal **(S2)** after the first impedance matching and the modified modulation signal **(SM')** compensated by a feedback signal **(ST)** to a target modified modulated radiofrequency carrier signal **(S2M'),**

a radiofrequency amplifier **(4)** configured and adapted to amplify a target modified modulated radiofrequency carrier signal **(S2M')** converted by the modulator **(11),**

an electrode **(6)** and a counter-electrode **(7),** wherein a RF current is generated between the electrode **(6)** and the counter-electrode **(7)** for capacitive coupled energy transfer;

a first impedance measuring means **(2)** configured and adapted to measure a first impedance **(I1)** caused by the device;

a first impedance matching means **(3)** configured and adapted to match the first impedance **(I1)** of the radiofrequency carrier signal **(S1)** measured by the first impedance measuring means **(2)** to a predetermined value;

a second impedance measuring means **(5)** configured and adapted to measure a second impedance **(I2)**;

a sensor **(12)** configured and adapted to detect a reflected or transmitted feedback signal **(ST);** and

at least one second impedance matching means **(8 or 8')**;

wherein the at least one second impedance matching means **(8 or 8')** is configured and adapted to match the second impedance **(I2)** measured by the second impedance measuring means **(5)** to the predetermined value.

In this embodiment, the second impedance **(I2)** refers to the second impedance of a target modulated radiofrequency carrier signal **(S3M')** after amplification, which is caused by the target **(17).**

In case the feedback signal **(ST)** needs to be amplified, the device of the present invention optionally further comprises a feedback amplifier **(14)** configured and adapted to amplify the feedback signal **(ST).** The feedback signal **(ST)** can be amplified to an amplified feedback signal **(ST2)** by means of a feedback signal amplifier **(14)** (Figures 19g, h and i).

In case the feedback signal amplifier **(14)** is present, the radiofrequency carrier signal generator **(1)** produces a radiofrequency carrier signal **(S1)** which is modulated by the modulation signal generator **(9)** to generate a target modulated radiofrequency carrier signal, the target modified modulated radiofrequency carrier signal **(S2M')** is amplified by the amplifier **(4)** and directed to a target, the impedance **(I2)** of the target modified modulated radiofrequency carrier signal **(S3M')** after the amplification is compensated by the second impedance matching means **(8 or 8')** after the amplification and the sensor **(12)** receives a feedback signal **(ST)** from the target that is directed to the feedback amplifier **(14),** wherein the feedback signal is amplified by the feedback amplifier to and modulates the carrier signal to generate a target modified modulated carrier signal. The impedance **(I2)** of the target modified modulated carrier signal is compensated by the second impedance matching means.

Optionally, signal checking unit (power/current sensor) **(15),** comparator **(13)** to a reference signal $(P_a(t))$ and reference signal $(P_a(t))$ are introduced into the inventive device which may serve for the fine-tuning of the feedback and the modulation as shown in Figure 19, h and i.

It is also possible to combine the function of two or more parts of the inventive hyperthermia device. For example, the modulation signal generator **(9)** and the feedback amplifier **(14)** can be combined so that amplification and modulation is performed by one part of the device. In such a case the modulation signal generator **(9)** could also amplify the feedback signal and also modulate the feedback signal in order to generate the modified modulation signal **(SM').**

In one embodiment, the inventive device of the present invention has a sensor used to detect the feedback signal from the target area and this information is used to adjust the amplitude for an optimized modulation/demodulation process, the frequency spectrum (preferably pink noise) of the modulation signal and modulate the carrier frequency emitted by the signal source via a feedback loop. The sensor detects the standing wave ratio (SWR). This ratio measures the proper matching of the RF energy which is the ratio between the sum and the difference of the voltages of the transmitted and reflected signals. $\left( SWR = \dfrac{V_{forwarded} + V_{reflected}}{V_{forwarded} - V_{reflected}} \right).$

The feedback sensor can be placed before the target tissue, or after the target. For example, the sensor can be situated between the amplifier and the target or the sensor can be situated between the target and the feedback amplifier. However, the modulation of the signal must occur before the target.

Also in this embodiment the presence of the feedback amplifier is preferred but not necessarily required if the feedback signal is strong enough and does not need to be amplified.

The modulated radiofrequency carrier signal as well as the target modified modulated radiofrequency carrier signal is applied to the target by capacitive coupling and not by radiation or radiative coupling. The capacitive coupling is done between an electrode and a counter electrode by forcing RF-current between them. That means, the RF current runs between both electrodes.

The RF current running between the electrodes has the advantage that it finds the target area by itself and does not require any focusing. Furthermore the electric field generated between the conductive electrodes induces apoptosis and kills, for instance, cancer cells by apoptosis and not by necrosis like the radiative coupling does which also kills healthy cells and mostly kills healthy cells when the radiative treatment is not focused well.

This radiofrequency (RF) hyperthermia device is designed for matching increasing the selectivity of the hyperthermia treatment by optimized double impedance matching.

In contrast, in case of the device according to the present invention, the selection is made on the cellular level, it is automatic, due to using the cellular differences between healthy and tumorous or cancerous cells. This is why the metabolic rate differs between the healthy or normal cells and the tumorous or cancerous cells by their ionic environment and so by their impedance. Therefore it is also very important that due to this fact the device of the present invention is also useful to treat cancer metastases and not only solid tumors, since the state of the art devices need to focus their generated heat on a macroscopic or larger area and the inventive device can also kill singles cancer cells and cancer metastases due to a differentiation of the cells on a microscopic level.

Thus due to the use of an electric field (near field approximation) by the inventive RF hyperthermia device, where the electric field makes the effect at the cellular level (membrane distortion), the present device is superior to the common devices, which apply the radiative solution uses the Poynting vector (a vector product or magnetic and electric fields) which are only useful to generate only heating.

Moreover the present inventive device uses amplitude modulation and not phase as the common devices of the state of the art do.

## Electrode

The inventive hyperthermia device uses capacitive coupling between the electrodes and RF current which also runs through the target tissue of the patient while the body part of the patient between the electrodes acts like a dielectric material wherein the target tissue is heated by Joule heat ($Q=I^2R$) generated by conversion of the current flow through the target tissue into heat as well as by the potential difference used for an electric field effect.

Selectivity of the generation of heat mostly within the target tissue or the diseased tissue and not the healthy tissue is achieved by using conductivity differences of the healthy tissue in regard to the diseased or target tissue. The target tissue such as a malignant tumor tissue has a higher complex or overall conductivity (admittance) than healthy tissue and consequently has a higher absorption rate of the current going through it in comparison to healthy or normal tissue so that the Joule heat is mostly generated when the current passes the target tissue.

The hyperthermia device of the present invention can be an electric field coupled energy transfer device (capacitive coupling device), a magnetic field coupled energy transfer device (inductive coupling device), or a radiative energy transfer device (radiative coupling or antenna array device). Preferably the radiofrequency hyperthermia device of the present invention is an electric field coupled energy transfer hyperthermia device (capacitive coupling device).

In one embodiment, the electrode (6) or the counter-electrode (7) of the inventive radiofrequency hyperthermia device is configured and adapted to any shape of the target (17).

In another embodiment, the second impedance matching means (8 or 8') of the inventive radiofrequency hyperthermia device is integrated into the electrode (6) or the counter-electrode (7).

The electrode can be a conventional bolus electrode where an active electrode is partnered with a counter electrode and the target tissue is placed between the active electrode and the counter electrode.

Alternatively, the electrode can be a flexible non-bolus type electrode. The flexible electrode can be in the form of a belt or bandage with lightning fastener. For example it could be a belt-like shape having paired electrodes, for example, with one pair or two pairs of capacitive electrodes. Instead of a coated flexible carrier or a coated flexible material a conductive metallic net or a conductive metallic network can be used, manufactured of at least one conductive metal electrode material. Such metallic nets or metallic networks preferably do not comprise any backbone such as a polymeric network structure. The metallic net or network is preferably a woven structure of metallic fibres having very similar properties as the coated flexible material such as a coated textile. The conductive metallic net or network is flexible, allows water and other fluids as well as gases to move through, can be folded without negative effect concerning conductivity and is able to cover uneven, fractal and/or percolative surfaces. Consequently, all kind of metallic nets and networks having the afore-mentioned properties of the conductively coated materials such as the conductively coated textiles are useful within the inventive electromagnetic energy transfer means. The conductive metal coating is a multilayer coating. Preferably, one of the layers is silver which has a good antibacterial effect and provides for good radiofrequency (RF) conductivity. Moreover, silver has an anti-odour effect together with moderate anti-perspiration activity. Therefore silver is preferred for cosmetic, medical and well-being applications. However, other conductive metal coatings can be used also. The resulting conductive metal coated flexible material is still sufficiently porous to allow for the exchange of heat and fluids.

These electrodes are an important part of the device according to the present invention. The belt electrodes or bandage electrodes are flexible, expandable, elastic and/or stretchable, can be provided in any desired size and can be easily used. The complete belt electrode or bandage electrode can form the conductive part of the electrode or only parts of the belt electrode or bandage electrode can form the conductive area of the electrode. These conductive parts or conductive areas can be made in any desired arrangement, shape, number and size. Preferably, also the conductive parts or conductive areas of the belt electrode or bandage electrode are flexible, expandable, elastic and/or stretchable so that they can adhere tightly to the area of the body which should be treated.

Instead of a coated flexible, elastic or stretchable carrier or a coated flexible material a conductive metallic net or a conductive metallic network manufactured of at least one conductive metal electrode material can be used. Such metallic nets or metallic networks do preferably not comprise any backbone such as a polymeric network structure. The metallic net or network is preferably a woven structure of metallic fibres having very similar properties as the coated flexible material such a coated textile. The conductive metallic net or network is flexible, elastic or stretchable, allows water and other fluids as well as gases to move through it, can be folded without negative effect concerning conductivity and is able to cover uneven, fractal and/or percolative surfaces. Consequently, all kind of metallic nets and networks having the afore-mentioned properties of the conductively coated materials such as the conductively coated textiles are useful within the inventive electromagnetic energy transfer means.

Percolation is a phenomenon occurring when a volume of surface is filled up by linear connection possibilities which could find special linear connection pathways between the ends. For example, a typical percolative device is the well-known carbon-powder microphone the conduction of which was changed by the pressure on the membrane, because more percolative pathways in the powder were created by the pressure on the powder. The polymerization process shows also a percolation phenomenon, and presently the internet network can also be described with such a term. The coating of a textile (or any fibre-woven or non-woven sheets) definitely shows a percolation problem: The conduction path between the two electrodes could be various zig-zags in the sheet, and its conductivity depends on the number of the connection zig-zags (the degree of percolation).

The carrier or support or material and especially the solid carrier or solid support or solid material is made of plastic, polymers or natural substances such as biopolymers, is coated with a conductive material such as a conductive metal or metal alloy. Moreover, said coated carrier or coated support or coated material is porous and allows a liquid to pass through said carrier or support or material. Furthermore, the coated carrier or coated support or coated material is flexible, i.e. does not have a definite or predefined shape and is able to follow the uneven curvatures of the human or animal body or any cavity containing liquids, fluids, gas or solid substances which should be heated up.

The term "porous" as used herein refers to the ability that the coated carrier or coated support or coated material allows water and any gas to move through said coated carrier or coated support or coated material. The pore size can be up to 0.1 mm or even larger.

A porous textile is suitable as the flexible material or flexible, elastic or stretchable carrier or flexible, elastic or stretchable support. However any porous material having flexibility similar to the flexibility of a woven or non-woven textile could also be used in the present invention. Thus any kind of textile, woven textile, non-woven textile and even non-textile material is suitable as flexible porous material. Such a flexible porous material can also be named as a flexible porous solid support or flexible porous solid carrier. Such materials, carrier or supports are not limited by a specific shape and have the consistency and/or texture of a piece of textile or a piece of fabric or drapery. Consequently, all known natural and artificial materials such as polyamide (Nylon®), poly-$\varepsilon$-caprolactone, poly-para-dioxanones, polyanhydrides, polyhydroxymethacrylates, fibrin, polyetherester, PEG, poly(butylene terephthalates), polycarbonates, poly(N-vinyl)-pyrrolidone, polyvinylalcohols, polyesteramides, polyethyleneoxide, polypropyleneoxide, polyurethanes, fibrinogen, starch, collagen, zein, casein, ß-cyclodextrins, polyacrylates, polyacrylamide, polyimides, polyethylene, polypropylene, polytetrafluoroethylene, fluorosilicones, rayon, polysulphones, silicones, polysiloxanes, polyvinyl halogens and copolymers or mixtures of these substances can be used.

Preferred are materials, carrier or supports such as these mentioned before which provide a good adhesion for the metallic coating. Also preferred are materials, carrier or supports which are manufactured of or which consist of a plurality of single fibres like a woven textile wherein one set of the single fibres extends along the complete length of the textile more or less in a substantially parallel manner while the other set of fibres is arranged in a substantially parallel manner diagonal to the first set of fibres. Thus fibres having a length which is similar to the length of the textile comprising said fibres are preferred.

In a further preferred embodiment of the present invention the single fibres of the material, carrier or support are coated like a tube which means not only a part of the surface of the fibre is coated rather the coating is applied all around the fibre.

Moreover it is preferred that such material, carrier or support is percolative and/or fractal or has a percolative and/or fractal structure without any discontinuity between the in and out cables of the electromagnetic energy transfer means. In other words, the metallic nets or the coated carriers are such flexible that they are able to cover uneven, fractal or percolative surfaces or they are able to follow the structure of uneven, fractal or percolative surfaces.

The conductive metal coating is a multilayer coating. Preferably, one of the layers is silver which has a good antibacterial effect and provides for good radiofrequency (RF) conduction. Moreover, silver has an anti-odour effect together with moderate anti-perspiration activity. This makes silver preferred for cosmetic, medical and well-being applications.

However, other conductive metal coatings can also be used. The resulting conductive metal coated flexible material is still sufficiently porous to allow for the exchange of heat and fluids. Preferably the electroless (autocatalytic) deposition of the conductive metal coating is used which is one of the most frequently used methods for fabricating coatings for corrosion- and wear-resistant purposes. The term "electroless coating" or "electroless deposition" refers to the building of a metallic coating from an electrolyte by way of immersion. Metallization of any nonconductive materials (e.g. plastics) occurs always (at least at the beginning) in such a chemical process. It is autocatalytic, using platinum or palladium salts to fix the first clusters on the surface "dangling" bonds.

The textile can be coated by extra plastic layer for galvanic isolation. This should preferably not be a continuous layer, but a layer on the fibres only in order to keep the porous structure free and open. If the device is double isolated, direct metallic contact could be used. The multilayer structure coats the fibres co-axially and perfectly continuous. If the metal layer-making technology is dipping electroless process then the crossing of individual fibres could be also perfectly coated individually fibre by fibre, if it is galvanic, then the crossings could be coated only as a cross, not keeping the individual fibre co-axial structure. The plastic coating, however, has to be dipping with such surface tension of the bath, which does not allow the isolation of the metallic coated fibres at their crossing, only coats their outer surface, however the coating does not fill up the pores, so the material remains porous.

Ideally, treatment electrodes or applicators are used that are suitable for application to all parts of the human body. For this reason there is a need for a flexible electrode/applicator that can conform to the contours of the body. The coated flexible carrier or the flexible metallic net acts as an electrode for the treatment of a large and/or uneven, fractal and/or percolative surface while these carriers or metallic nets can easily be fixed on said surface via a belt or bandage, or the like.

The inventive flexible electric field capacitive coupled electrode of the present invention is able to fulfil this requirement, i.e. to be conductive, flexible, foldable, porous and able to cover uneven, fractal and/or percolative surfaces smoothly like a cloth following the contour of the surface. The flexible electrode applicator is formed from a conductive metal coated flexible material or a metallic network that can be folded or formed freely even into a cylinder-like shape. Thus the inventive electrode can conform to gradual and sharp curvatures. For example, in the case of sharp curvatures it can easily conform to the shape of a limb by wrapping around the limb or can conform to the shape of the skull. Alternatively, in the case of gradual curvatures it can conform to the shape of the torso.

Also the inventive flexible electromagnetically coupled electrode applicator is lightweight and therefore can be produced to cover a large area, such as the torso, without causing discomfort to the patient due to excessive weight of the electrode applicator. This allows for the treatment of large areas in a single treatment session. Also the flexibility of the energy transfer means allows for a good contact between the applicator and a large application area, for example, the torso.

The inventive flexible electromagnetically coupled electrode applicator is also porous. This allows for natural cooling of the treatment area due to exchange of heat through the energy transfer means via convection. Also a simple external air cooling system, for example a directed air flow from a fan, can be used to cool the application area to prevent burning and maintain patient comfort. As a result there is no requirement for a complicated fluid cooling system as is the case in conventional bolus electrodes. This allows for a simple and lightweight construction that is suitable for home use by a patient.

Additionally, an extra air or fluid cooling can optionally be introduced to the electrodes. An air-cooling by conventional ventilation from a definite distance could be applied. The fluid cooling is another optional solution, but the simplest is a wet textile tissue covering the electrodes. The fluid itself and the evaporation of the fluid cause intensive cooling. This can be combined with air-cooling to generate more intensive evaporation, using the necessary latent heat.

**Target**

In one embodiment, the target **(17)** of the radiofrequency hyperthermia device is a tissue, an organ, a part or hole of a human or an animal body.

The term "target" as used herein refers to the object (i.e. patient, human or animal) to be treated with hyperthermia or oncothermia. The inventive hyperthermia device is especially useful for the treatment of cancer, solid tumors but also cancer metastases.

Thus, the hyperthermia device of the present invention can be used to selectively treat a localised target site wherein the localised target site is selected from tumour tissues and muscle tissue, or organs, such as for example liver, lung, heart, kidney, spleen, brain, ovary, uterus, prostate, pancreas, larynx, the gastrointestinal tract, and the gynaecological tract.

The tumour tissue can be selected from adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma and tongue cancer.

Particularly suitable for treatment are, for example, astrocytomas, glioblastomas, pancreatic cancer, bronchial cancer, breast cancer, colorectal cancer, ovarian cancer, gastric cancer, laryngeal cancer, malignant melanoma, oesophageal cancer, cervical cancer, liver cancer, bladder cancer, and renal cell cancer

The hyperthermia device of the present invention can be used in combination with chemotherapy treatment with cytostatic and/or cytotoxic drugs. Example of some cytostatic and/or cytotoxic drugs are actinomycin D, aminoglutethimide, amsacrin, anastrozol, antagonists of purine and pyrimidine bases, anthracycline, aromatase inhibitors, asparaginase, antiestrogenes, bexaroten, bleomycin, buselerin, busulfan, camptothecin derivates, capecitabin, carboplatin, carmustine, chlorambucil, cisplatin, cladribin, cyclophosphamide, cytarabin, cytosinarabinoside, alkylating cytostatics, dacarbacin, dactinomycin, daunorubicin, docetaxel, doxorubicin (adriamycin), doxorubicin lipo, epirubicin, estramustine, etoposid, exemestan, fludarabin, fluorouracil, folic acid antagonists, formestan, gemcitabin, glucocorticoides, goselerin, hormones and hormone antagonists, hycamtin, hydroxy urea, idarubicin, ifosfamid, imatinib, irinotecan, letrozol, leuprorelin, lomustin, melphalan, mercaptopurine, methotrexate, miltefosin, mitomycine, mitosis inhibitors, mitoxantron, nimustine, oxaliplatin, paclitaxel, pentostatin, procarbacin, tamoxifen, temozolomid, teniposid, testolacton, thiotepa, thioguanine, topoisomerase inhibitors, topotecan, treosulfan, tretinoin, triptorelin, trofosfamide, vinblastine, vincristine, vindesine, vinorelbine, antibiotics with cytotoxic activities. All present and future cytostatics or other medicaments including gene therapy could be applied.

When used for treatment of inflammatory conditions the hyperthermia device of the present invention can be used in combination with an anti-inflammatory drug treatment such as a non-steroidal anti-inflammatory drug (NSAID), for example, alcofenac, aceclofenac, sulindac, tolmetin, etodolac, fenopren, thiaprofenic acid, meclofenamic acid, meloxicam, tenoxicam, lornoxicam, nabumetone, acetaminophen, phenacetin, ethenzamide, sulpyrine, mefanamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, piroxicam, epirizole, tiaramide hydrochloride, zaltoprofen, gabexate mesilate, camostat mesilate, ulinastatin, colchicine, probenecid, sulfinpyrazone, benzbromarone, allopurinol, salicylic acid, atropine, scopolamine, levorphanol, ketorolac, tebufelone, tenidap, clofezone, oxyphenbutazone, prexazone, apazone, benzydamine, bucolome, cinchopen, clonixin, ditrazol, epirizole, fenoprofen, floctafenin, glaphenine, indoprofen, niflumic acid and suprofen, or with a steroidal anti-inflammatory drugs, for example, dexamethasone, hexestrol, methimazole, betamethasone, triamcinolone, fluocinonide, prednisolone, methylprednisolone, hydrocortisone, fluorometholone, beclomethasone dipropionate, estriol, clobetasol, diflorasone diacetate, halbetosal propionate, amicinonide, desoximetasone, halcinonide, mometasone furoate, fluticasone propionate, flurandrenolide, clocortalone, predincarbate, aclometasone dipropionate and desonide.

## A method for matching an impedance of a radiofrequency carrier signal

The present invention also includes a method for matching an impedance of a radiofrequency carrier signal of the radiofrequency hyperthermia device comprising the steps of:

(a) providing a radiofrequency carrier signal **(S1)** by means of a radiofrequency carrier generator **(1);**

(b) measuring an impedance **(I1)** of the radiofrequency carrier signal **(S1)** by means of a first impedance measuring means **(2);**

(c) matching the impedance **(I1)** of the radiofrequency carrier signal **(S1)** by means of a first impedance matching means **(3)** to obtain an radiofrequency carrier signal **(S2)** obtained after the first impedance matching;

(d) amplifying the radiofrequency carrier signal **(S2)** obtained after the first impedance matching by means of a radiofrequency amplifier **(4)** to obtain an amplified radiofrequency carrier signal **(S3);**

(e) measuring an impedance **(I2)** directly at a target **(17)** by means of a second impedance measuring means **(5);**

(f) compensating the impedance **(I2)** by means of at least one second impedance matching means **(8** or **8');**

(g) directing the impedance matched carrier signal to an electrode **(6);** and

(h) generating a RF current between the electrode **(6)** and a counter-electrode **(7)** for capacitive coupled energy transfer.

In one embodiment, the step (d) of the above-mentioned method can be replaced with the following steps (d1) and (d2):

(d1a) modulating the radiofrequency carrier signal **(S2)** with a modulation signal **(SM)** provided by means of a modulating signal generator **(9)** to obtain a modulated radiofrequency carrier signal **(S2M);**

(d2a) amplifying the modulated radiofrequency carrier signal **(S2M)** by means of a radiofrequency amplifier **(4)** to obtain a modulated radiofrequency carrier signal **(S3M)** after amplification.

Optionally, a further step (d1a') can be performed between the step (d1a) and (d2a) as follows:

(d1a') controlling the modulated radiofrequency carrier signal **(S2M)** by means of a signal checking unit **(15).**

In another embodiment, the modulation signal can be compensated by a feedback signal **(ST),** and the steps (d1a) and (d2a) of the above-mentioned method can be replaced with the alternative steps (d1b) and (d2b):

(d1b) modulating the radiofrequency carrier signal **(S2)** with a modified modulation signal **(SM')** provided by means of a modulating signal generator **(9)** to obtain a target modified modulated radiofrequency carrier signal **(S2M');**

(d2b) amplifying the target modified modulated radiofrequency carrier signal **(S2M')** by means of a radiofrequency amplifier **(4)** to obtain a target modified modulated radiofrequency carrier signal **(S3M')** after amplification.

Optionally, a further step (d1 b') can be performed between the step (d1 b) and (d2b) as follows:

(d1b') controlling the target modified modulated radiofrequency carrier signal **(S2M')** by means of a signal checking unit **(15).**

In this embodiment, the method further comprises the following steps (i) and (j) after the step (h):

(i) detecting a feedback signal **(ST)** received by means of a sensor **(12);**
(j) sending the feedback signal **(ST)** received to the modulation signal generator **(9)** for providing a modified modulation signal **(SM')** compensated by the feedback signal in the step (d1 b);

wherein the step (d) is replaced with the following steps (d1 b) and (d2b):.

(d1 b) modulating the radiofrequency carrier signal **(S2)** with a modified modulation signal **(SM')** provided by means of a modulating signal generator **(9)** to obtain a target modified modulated radiofrequency carrier signal **(S2M');**

(d2b) amplifying the target modified modulated radiofrequency carrier signal **(S2M')** by means of a radiofrequency amplifier **(4)** to obtain a target modified modulated radiofrequency carrier signal **(S3M')** after amplification.

Optionally, the feedback signal **(ST)** can be amplified and thus the method further comprises the following steps (i), (i') and (j) after the step (h):

(i) detecting a feedback signal **(ST)** received by means of a sensor **(12);**
(i') amplifying the feedback signal **(ST)** by means of an amplifier **(14)** to an amplified feedback signal **(ST2),**
(j') sending the amplified feedback signal **(ST2)** received from the amplifier **(14)** to the modulation signal generator **(9)** for providing a modified modulation signal **(SM')** compensated by the feedback signal in the step (d1 b).

In general, the feedback signal **(ST)** is received from the target.

In case the steps (i) and (j) are performed, the step (d) is replaced with any one of the following steps:

- step (d1a) → step (d2a);
- step (d1a) → step (d1a') → step (d2a);
- step (d1b) → step (d2b);
- step (d1 b) → step (d1b') → step (d2b);

Therefore, the steps (d1a), (d1a'), (d2a), (d1b), (d1b'), (d2b), (i), (i'), (j) and (j') can be replaced and/or combined with the steps (a)-(h) as follows:

- step (a) → step (b) → step (c) → step (d1a) → step (d2a) → step (e) → step (f) → step (g) → step (h);
- step (a) → step (b) → step (c) → step (d1a) → step (d1a') → step (d2a) → step (e) → step (f) → step (g) → step (h);
- step (a) → step (b) → step (c) → step (d1 b) → step (d2b) → step (e) → step (f) → step (g) → step (h) → step (i) → step (j);
- step (a) → step (b) → step (c) → step (d1b) → step (d1b') → step (d2b) → step (e) → step (f) → step (g) → step (h) → step (i) → step (j);
- step (a) → step (b) → step (c) → step (d1 b) → step (d2b) → step (e) → step (f) → step (g) → step (h) → step (i) → step (i') → step (j'); or
- step (a) → step (b) → step (c) → step (d1b) → step (d1b') → step (d2b) → step (e) → step (f) → step (g) → step (h) → step (i) → step (i') → step (j').

Therefore, one embodiment of the present invention is a method for matching an impedance of a radiofrequency carrier signal of the radiofrequency hyperthermia device comprising the steps of:

(a) providing a radiofrequency carrier signal **(S1)** by means of a radiofrequency carrier generator **(1)**;

(b) measuring an impedance **(I1)** of the radiofrequency carrier signal **(S1)** by means of a first impedance measuring means **(2)**;

(c) matching the impedance **(I1)** of the radiofrequency carrier signal **(S1)** by means of a first impedance matching means **(3)** to obtain an radiofrequency carrier signal **(S2)** obtained after the first impedance matching;

(d1a) modulating the radiofrequency carrier signal **(S2)** with a modulation signal **(SM)** provided by means of a modulating signal generator **(9)** to obtain a modulated radiofrequency carrier signal **(S2M)**;

(d2a) amplifying the modulated radiofrequency carrier signal **(S2M)** by means of a radiofrequency amplifier **(4)** to obtain a modulated radiofrequency carrier signal **(S3M)** after amplification;

(e) measuring an impedance **(I2)** directly at a target **(17)** by means of a second impedance measuring means **(5)**;

(f) compensating the impedance **(I2)** by means of at least one second impedance matching means **(8 or 8')**;

(g) directing the impedance matched carrier signal **(S4M)** to an electrode **(6)**; and

(h) generating a RF current between the electrode **(6)** and a counter-electrode **(7)** for capacitive coupled energy transfer.

In one embodiment of the present invention, a method for matching an impedance of a radiofrequency carrier signal of the radiofrequency hyperthermia device comprises the steps of:

(a) providing a radiofrequency carrier signal **(S1)** by means of a radiofrequency carrier generator **(1)**;

(b) measuring an impedance **(I1)** of the radiofrequency carrier signal **(S1)** by means of a first impedance measuring means **(2)**;

(c) matching the impedance **(I1)** of the radiofrequency carrier signal **(S1)** by means of a first impedance matching means **(3)** to obtain an radiofrequency carrier signal **(S2)** obtained after the first impedance matching;

(d1a) modulating the radiofrequency carrier signal **(S2)** with a modulation signal **(SM)** provided by means of a modulating signal generator **(9)** to obtain a modulated radiofrequency carrier signal **(S2M)**;

(d1a') controlling the modulated radiofrequency carrier signal **(S2M)** by means of a signal checking unit **(15)**;

(d2a) amplifying the modulated radiofrequency carrier signal **(S2M)** by means of a radiofrequency amplifier **(4)** to obtain a modulated radiofrequency carrier signal **(S3M)** after amplification;

(e) measuring an impedance **(I2)** directly at a target **(17)** by means of a second impedance measuring means **(5)**;

(f) compensating the impedance **(I2)** by means of at least one second impedance matching means **(8 or 8')**;

(g) directing the impedance matched carrier signal **(S4M)** to an electrode **(6)**; and

(h) generating a RF current between the electrode **(6)** and a counter-electrode **(7)** for capacitive coupled energy transfer.

In one embodiment of the present invention, a method for matching an impedance of a radiofrequency carrier signal of the radiofrequency hyperthermia device comprises the steps of:

(a) providing a radiofrequency carrier signal **(S1)** by means of a radiofrequency carrier generator **(1)**;

(b) measuring an impedance **(I1)** of the radiofrequency carrier signal **(S1)** by means of a first impedance measuring means **(2)**;

(c) matching the impedance **(I1)** of the radiofrequency carrier signal **(S1)** by means of a first impedance matching means **(3)** to obtain an radiofrequency carrier signal **(S2)** obtained after the first impedance matching;

(d1b) modulating the radiofrequency carrier signal **(S2)** with a modified modulation signal **(SM')** provided by means of a modulating signal generator **(9)** to obtain a target modified modulated radiofrequency carrier signal **(S2M')**;

(d2b) amplifying the target modified modulated radiofrequency carrier signal **(S2M')** by means of a radiofrequency amplifier **(4)** to obtain a target modified modulated radiofrequency carrier signal **(S3M')** after amplification;

(e) measuring an impedance **(I2)** directly at a target **(17)** by means of a second impedance measuring means **(5)**;

(f) compensating the impedance **(I2)** by means of at least one second impedance matching means **(8 or 8')**;

(g) directing the impedance matched carrier signal **(S4M')** to an electrode **(6)**;

(h) generating a RF current between the electrode **(6)** and a counter-electrode **(7)** for capacitive coupled energy transfer;

(i) detecting a feedback signal **(ST)** received by means of a sensor **(12)**; and

(j) sending the feedback signal **(ST)** received to the modulation signal generator **(9)** for providing a modified modulation signal **(SM')** compensated by the feedback signal in the step (d1 b).

In another embodiment of the present invention, a method for matching an impedance of a radiofrequency carrier signal of the radiofrequency hyperthermia device comprises the steps of:

(a) providing a radiofrequency carrier signal **(S1)** by means of a radiofrequency carrier generator **(1)**;

(b) measuring an impedance **(I1)** of the radiofrequency carrier signal **(S1)** by means of a first impedance measuring means **(2)**;

(c) matching the impedance **(I1)** of the radiofrequency carrier signal **(S1)** by means of a first impedance matching means **(3)** to obtain an radiofrequency carrier signal **(S2)** obtained after the first impedance matching;

(d1b) modulating the radiofrequency carrier signal **(S2)** with a modified modulation signal **(SM')** provided by means of a modulating signal generator **(9)** to obtain a target modified modulated radiofrequency carrier signal **(S2M')**;

(d2b) amplifying the target modified modulated radiofrequency carrier signal **(S2M')** by means of a radiofrequency amplifier **(4)** to obtain a target modified modulated radiofrequency carrier signal **(S3M')** after amplification;

(e) measuring an impedance **(I2)** directly at a target **(17)** by means of a second impedance measuring means **(5)**;

(f) compensating the impedance **(I2)** by means of at least one second impedance matching means **(8 or 8')**;

(g) directing the impedance matched carrier signal **(S4M')** to an electrode **(6)**;

(h) generating a RF current between the electrode **(6)** and a counter-electrode **(7)** for capacitive coupled energy transfer;

(i) detecting a feedback signal **(ST)** received by means of a sensor **(12)**;

(i') amplifying the feedback signal **(ST)** by means of an amplifier **(14)** to an amplified feedback signal **(ST2)**; and

(j') sending the amplified feedback signal **(ST2)** received from the amplifier **(14)** to the modulation signal generator **(9)** for providing a modified modulation signal **(SM')** compensated by the amplified feedback signal **(ST2)** in the step (d 1 b).

In further embodiment of the present invention, a method for matching an impedance of a radiofrequency carrier signal of the radiofrequency hyperthermia device comprises the steps of:

(a) providing a radiofrequency carrier signal **(S1)** by means of a radiofrequency carrier generator **(1)**;

(b) measuring an impedance **(I1)** of the radiofrequency carrier signal **(S1)** by means of a first impedance measuring means **(2)**;

(c) matching the impedance **(I1)** of the radiofrequency carrier signal **(S1)** by means of a first impedance matching means **(3)** to obtain an radiofrequency carrier signal **(S2)** obtained after the first impedance matching;

(d1b) modulating the radiofrequency carrier signal **(S2)** with a modified modulation signal **(SM')** provided by means of a modulating signal generator **(9)** to obtain a target modified modulated radiofrequency carrier signal **(S2M')**;

(d1b') controlling the target modified modulated radiofrequency carrier signal **(S2M')** by means of a signal checking unit **(15)**;

(d2b) amplifying the target modified modulated radiofrequency carrier signal **(S2M')** by means of a radiofrequency amplifier **(4)** to obtain a target modified modulated radiofrequency carrier signal **(S3M')** after amplification;

(e) measuring an impedance **(I2)** directly at a target **(17)** by means of a second impedance measuring means **(5)**;

(f) compensating the impedance **(I2)** by means of at least one second impedance matching means **(8 or 8')**;

(g) directing the impedance matched carrier signal **(S4M')** to an electrode **(6)**;

(h) generating a RF current between the electrode **(6)** and a counter-electrode **(7)** for capacitive coupled energy transfer;

(i) detecting a feedback signal **(ST)** received by means of a sensor **(12)**; and

(j) sending the feedback signal **(ST)** received to the modulation signal generator **(9)** for providing a modified modulation signal **(SM')** compensated by the feedback signal in the step (d1 b).

In further embodiment of the present invention, a method for matching an impedance of a radiofrequency carrier signal of the radiofrequency hyperthermia device comprises the steps of:

(a) providing a radiofrequency carrier signal **(S1)** by means of a radiofrequency carrier generator **(1)**;

(b) measuring an impedance **(I1)** of the radiofrequency carrier signal **(S1)** by means of a first impedance measuring means **(2)**;

(c) matching the impedance **(I1)** of the radiofrequency carrier signal **(S1)** by means of a first impedance matching means **(3)** to obtain an radiofrequency carrier signal **(S2)** obtained after the first impedance matching;

(d1b) modulating the radiofrequency carrier signal **(S2)** with a modulation signal **(SM')** provided by means of a modulating signal generator **(9)** to obtain a target modified modulated radiofrequency carrier signal **(S2M')**;

(d1b') controlling the target modified modulated radiofrequency carrier signal **(S2M')** by means of a signal checking unit **(15)**;

(d2b) amplifying the target modified modulated radiofrequency carrier signal **(S2M')** by means of a radiofrequency amplifier **(4)** to obtain a target modified modulated radiofrequency carrier signal **(S3M')** after amplification;

(e) measuring an impedance **(I2)** directly at a target **(17)** by means of a second impedance measuring means **(5)**;

(f) compensating the impedance **(I2)** by means of at least one second impedance matching means **(8 or 8')**;

(g) directing the impedance matched carrier signal **(S4M')** to an electrode **(6)**;

(h) generating a RF current between the electrode **(6)** and a counter-electrode **(7)** for capacitive coupled energy transfer;

(i) detecting a feedback signal **(ST)** received by means of a sensor **(12)**;

(i') amplifying the feedback signal **(ST)** by means of an amplifier **(14)** to an amplified feedback signal **(ST2)**; and

(j') sending the amplified feedback signal **(ST2)** received from the amplifier **(14)** to the modulation signal generator **(9)** for providing a modified modulation signal **(SM')** compensated by the amplified feedback signal **(ST2)** in the step (d 1 b).

The advantages of the second impedance matching are proven in the experiments described in the examples 1 and 2.

In the experiment the main tuner was tuned on its own optimum (as usual in all the experiments, it is automatic) and an additional second tuning just near to the target is applied. OTM2 is the real-part adjustment (ohmic component) and OTM3 is the imaginary part component (inductive compensation). The *in vitro* experiments show that the important apoptotic cell death (in progress and late) are significantly higher in the case of additional (described in the application) adjustment of the impedance.

The *in vivo* experiments support the *in vitro* observations. This experiment shows that the secondary tuning boosts robustly the tumor-destruction which is more dependent on this extra tuning than from the additional forwarded energy. It is important not only in the point of view of the present application but in general use of the hyperthermia it has also an important message. Many time when the reflected power is large, they increase the forwarded one for obtaining the requested temperature. This experiment show, that the power increase is not such effective as the proper tuning, which reduces the reflected power, so the desired absorbed energy would be available at the constant forwarded power.

As discussed above, the first stage of *in vitro* experiments (OTM1) is the single tuning case, the OTM2 and OTM3 are the double tuning, The OTM4 is only for the low temperature, it has no real relevance to the tuning, it is only the check of the effects in low temperature. It shows that with double tuning the apoptosis is more than the control despite that the experiment was made in 37°C that is almost normothermia. It is remarkable.
In case of *in vivo* experiments we see the same. The setup 1 is the original single tuning, it has less tumor-destruction volume than all the other modified secondary tuning experiment (various secondary tuning with real part and imaginary part of the impedance were investigated.)

The reference signs used in the description and Figure 19 are defined as follows:

**(1):** a radiofrequency carrier signal generator, for example, oscillator which provides the selected frequency (preferably 13.56 MHz) by means of a fixed stable quartz-oscillator
**(2):** a first impedance detecting means
**(3)** a first impedance matching means, for example, a matching tuner in figure 15
**(4):** a radiofrequency amplifier
**(5):** a second impedance detecting means
**(6):** an electrode
**(7):** a counter-electrode
**(8):** a second impedance matching means, for example, a local compensation unit in figure 15
**(9):** a modulation signal generator
**(11):** a modulator
**(12)** a sensor (current / power) detecting the feedback signal of the target,
**(13):** optionally, a comparator to a reference signal ($P_a$(t) ) which controls the signal by comparison to the reference,
**(14):** a feedback signal amplifier which amplifies the feedback signal up to the desired level for further use,
**(15):** optionally, a signal checking unit (power/current sensor) which senses the signal-amplitude for control purposes,
**(17):** a target,
**(18):** a RF-ground, a ground level not necessarily identical with the general ground (earth-potential). This ground is modified by the respective potential distribution of the RF signal as a function of its wavelength.

**(x(t)):** an amplified and target-modified signal (modulated signal) which is responsible for the treatment in the target tissue,
**(I1):** a first impedance measured by means of a first impedance detecting means **(2)**
**(I2):** a second impedance measured by means of a second impedance detecting means **(5)**
**(S1):** a radiofrequency carrier signal generated by means of a radiofrequency carrier signal generator **(1)**
**(S2):** a radiofrequency carrier signal obtained after the first impedance matching by means of the first impedance matching means **(3)**
**(SM):** a modulation signal
**(SM'):** a modified modulation signal compensated by a feedback signal or by an amplified feedback signal
**(S2M):** a modulated radiofrequency carrier signal
**(S2M'):** a target modified modulated radiofrequency carrier signal
**(S3):** a radiofrequency carrier signal obtained after amplification by means of the amplifier **(4)**, i.e. an amplified radiofrequency carrier signal
**(S3M):** a modulated radiofrequency carrier signal obtained after amplification by means of the amplifier **(4)**
**(S3M'):** a target modified modulated radiofrequency carrier signal obtained after amplification by means of the amplifier **(4)**
**(S4):** a radiofrequency carrier signal obtained after the second impedance matching by means of the second impedance matching means **(8** or **8'**)

**(S4M):** a modulated radiofrequency carrier signal obtained after the second impedance matching by means of the second impedance matching means **(8** or **8')**

**(S4M'):** a target modified modulated radiofrequency carrier signal obtained after the second impedance matching by means of the second impedance matching means **(8** or **8')**

**(ST):** a feedback signal which carries the information of the actual treatment in a complex form and contributes to its control,

**(ST2):** a feedback signal amplified by means of the amplifier **(14)**

**($P_a(t)$):** optionally, a reference signal, as a stable signal for fixing signal levels.

## Description of the figures:

| | |
|---|---|
| Figure 1 | Impedance components in inductive (a) and capacitive (b) dominant circuits. |
| Figure 2 | The complex numbers can be handled like vectors, so their serial connection could be described with the vectorial addition. |
| Figure 3 | Combination an inductive and capacitive part with the same absolute value of imaginary part, then the system looks like a simple real-resistive one. This "compensation" would be the optimal. In this case there is no reflected power, no loss, all the energy is in the target. |
| Figure 4 | Impedance vectors, when the tumor/healthy tissue mass is fixed in the target (same electrode diameter on the same distances of electrodes). Smaller or dispersed tumor-size in the given volume lowers the $R_h$ and $X_h$, so $|Z_h|^2 = R_h^2 + X_h^2$ decreases, however $R_t$ and $X_t$, increases while so $|Z_t|^2 = R_t^2 + X_t^2$ increases. |
| Figure 5 | Compensation of reactance originates pure resistive (ohmic) resulting impedance. |
| Figure 6 | Vectorial summation for showing the resulting impedance. |
| Figure 7 | The resulting impedance of the tumor and its vicinity (a) and the compensation process as the turn of the resulting impedance into the horizontal. |
| Figure 8 | The compensation could be done by differ- |

ent processes, depending on the actual compensation element (a) It could be a solution by inductively over-compensated, and after it re-compensated by variable condenser (only resistance is constructed again) (b).

(a) The compensation of the impedance could be one by different impedances depending on the vector-components of the compensating impedance. This means that the tuning has a wide variability to solve the actual optimum. However, when we chose and fix the components of the tuner (the variability of ohmic and the imaginary parts), then the tuning has only one solution in the practice.

(b) When technically the inductive compensation could be solved with difficulties, then it has to be overcompensated, and with the much easier variable capacitor (which has much less dissipation than an induction) we may re-compensate the full system.

| | |
|---|---|
| Figure 9 | Shown is a target, like a patient reclining on a waterbed and having a water bolus electrode on his abdomen. The water bolus electrode and the water bed counter electrode are both connected to a radiofrequency generator. Consequently, the target is part of the condenser arrangement and it is evident that the target is part of the electric circuit. |
| Figure 10 | Various layers of impedances in the body in the targeted volume. |
| Figure 11 | Various layer-networks make different resulting impedances. |
| Figure 12 | The variable electrode impedance is certainly a controlling possibility of the resulting impedance. |
| Figure 13 | (a) Over-compensated resulting impedance for full compensation of the tumor-impedance, and (b) under-compensated resulting impedance for full compensation of the tumor-impedance. |
| Figure 14 | There are various impedances inside the body. The impedance vectors can be tacking in a line which I did in the figure. The consequent vector connects the start of the first and the end of the last vector components. Our aim is to make compensation, which prefers one of the vector-components in the chain of vectors to fix is a most |

ohmic (largest absorbed energy) position. For this the consequent vector will not be parallel with the ohmic axis, but has an angle with it promoting the parallelism of the vector (tumor impedance), which is preferred by optimal tuning.

Figure 15    The compensation positions a) ground-side; b) RF-hot-side. Note, the ground independent solution is also logically available on these ways.

Figure 16    Tuning the impedance by positioning a core material.

Figure 17    Tuning the impedance by connecting in various lengths of the coil.

Figure 18    Over-tuned and re-compensated circuit by changeable condenser.

Figure 19    (a) an inventive hyperthermia device of the present invention; (b) an inventive hyperthermia device of which a first impedance matching mean is integrated into a radiofrequency carrier signal generator; (c) an inventive hyperthermia device having at least one second impedance matching means integrated into an electrode and/or a counter-electrode; (d) and (e) various types of inventive hyperthermia device having a modulator; (f), (g), (h) and (i) various types of inventive hyperthermia device having a modulator and a feedback signal modulator.

Figure 20    The experimental setups of the hyperthermia treatments mEHT (A) and WHT (B)

Figure 21    Representative temperature measurement graphs during mEHT and WHT

Figure 22    The measuring setup. The resistivity is changeable by the immersion of the platinum electrode, and measured with a special data-collection system, developed for these experiments (photo far left). The data-collection box (left side on the photo in the middle) and the inductive tuning unit, the photo on right shows the switching possibility of the inductive values.

Figure 23    The microscopic images: a) Control (CTRL), b.) matched with balloon transformer, which is the conventional impedance transformation in state of art, (OTM1), c.) matched with partial immersion of the electrode, which modifies the real-part of the complex impedance, (OTM2), d. matched with induction (coil) which modifies the imaginary part of the impedance, (OTM3), e.) same as d.) only the heating up to 37°C, (OTM4), while all others were heated to 42°C.

Figure 24    The results of experiments (three repetitions) measured by flow-cytometry 3h post treatment using Annexin-PI apoptosis kit. The abbreviations: CTRL = control, no treatment is applied; OTM1 = matched with balloon transformer, which is the conventional impedance transformation in state of art; OTM2 = matched with partial immersion, which modifies the real-part of the complex impedance; OTM3 = matched with induction which (coil) which modifies the imaginary part of the impedance; OTM4 = same as OTM3, only the heating up to 37°C, while all others were heated to 42°C.

Figure 25    The results of experiments (three repetitions) measured by flow-cytometry 3h post treatment using Annexin-PI apoptosis kit. The columns on the upper panel are the control, (samples which were non treated at all), the less inductive matching, and the proper inductive matching. The matching was controlled by network analyzer, (Vector Impedance Antenna Analyzer, SARK-110, Melchor Varela, Spain) and the actual matching measurements are shown on the photos b) and c)-b) belongs to the middle column on the upper panel, while c) shows the matching of the right column

Figure 26    a) SETUP-1. Original electrode setup (the treating pad grounded directly to the RF ground), electrode gel (Aquasonic 100, Parker Laboratories Inc. USA) + household polyethylene (PE) foil. b) After the electrode was placed in the proper position onto the tumor, but before the treatment was started, a network analyzer (Sark-110) was connected to the input of the in vivo applicator to measure the impedance parameters (real and imaginary parts) and S11, Forward Reflection (input match - impedance) parameter..

Figure 27    a) SETUP-2: Modified electrode grounding (the treating pad grounded via the variable inductance) applied electrode gel + PE foil measured by SHARK-110. In this setup the impedance was adjusted to form a resonant LC circuit, measured the S11 (Forward Reflection (input match - impedance)) parameter. b) the network analyser was used to "tune" the electrode-tumor system.

Figure 28    a) SETUP-3: Modified electrode grounding (the treating pad grounded via the variable inductance) without electrode gel + PE foil, the textile electrode was placed directly to the skin surface. In this setup the impedance was adjusted to form a resonant LC circuit. b) the network analyzer

Figure 29    a) SETUP-4: Modified electrode grounding (the treating pad grounded via the variable inductance) without electrode gel + PE foil, the textile electrode was placed directly to the skin surface. In this setup the impedance was adjusted to form a resonant LC circuit. Intensive active cooling by sponge moisture with water and placed on the top of the electrode, was applied on the tumor surface enabling to deliver higher dose RF energy (instead the average 2kJ we applied 2.5kJ in these cases). b) the network analyzer

Figure 30    shows the mice from SETUP-1 (mice 1-3) and from SETUP-2 (mice 4-6) immediately after their sacrifice. The "NO COMP" means "no compensation", which is SETUP-1.

Figure 31    Results of SETUP-1: When the ohmic component is also modified, the tuner stability and the complete matching are better.

Figure 32    Results of SETUP-2: Further variation of the ohmic component is stable, but the well is narrower, the stability against movements of the animal would be less.

Figure 33    Results of SETUP-3: It is clear, that the 3 of setup 1, when the absorbed energy is the highest compared to the constant forwarded power, has the internal distortion of the tumor. The others have external starting from the side of the electrode, but larger area than the 3$^{rd}$ one. This setup is single tuner (no modification) and shows that the relative absorbed energy is not decisional.

Figure 34    Results of SETUP-4: This setup is on the small temperature, which does not show specular tumor destruction, but the apoptotic processes and active. It is important here again, that not the overall value of the absorbed energy is the primary factor of the effect.

**EXAMPLES**

**Example 1 *In vitro* experiment**

During the *in vitro* experiments the compensation of the capacitor of the flask-glasses and the variation of the resistivity were measured. The compensation was done by balloon-transformer, and by the impedance matching with an inductivity.

U937, a human myelomonocytic lymphoma cell line from Human Sciences Research Resource Bank (Japan Human Sciences Foundation, Tokyo, Japan), was used for the experiments. The cells were grown in RPMI 1640 culture medium supplemented with 10% heat-inactivated foetal bovine serum (FBS) at 37°C in humidified air with 5% $CO_2$. Cells were subcultured every second day, and used for the experiments in their log phase. Cells were treated at a density of $10^6$ cells/mL, in a total volume of 8mL.

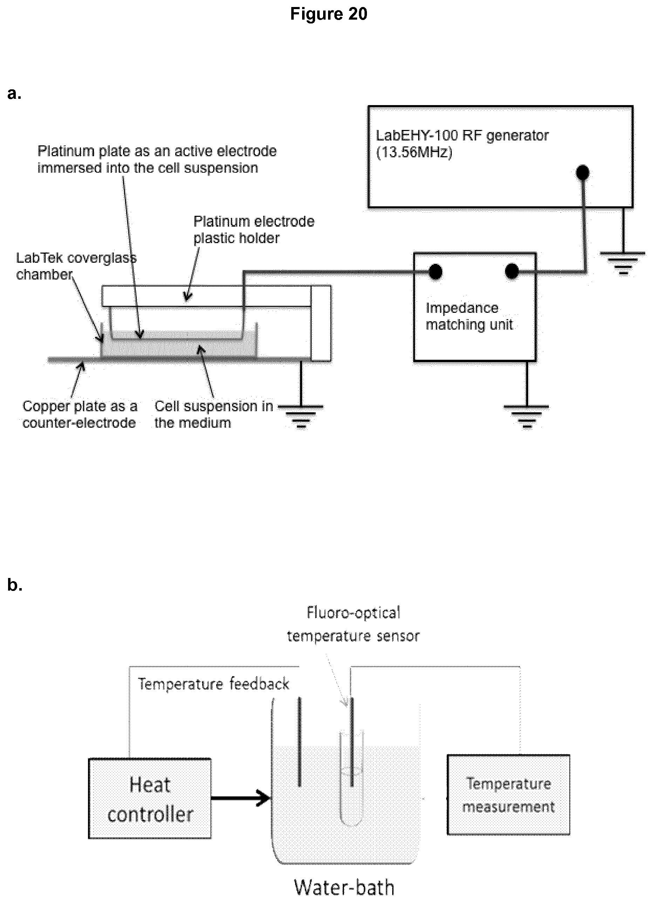

Here, 8mL of U937 cell suspension was used in both of the treatment objects. In the mEHT treatment process, the suspension was pipetted into a coverslip-bottomed slide-flask (Nunc™ Lab-Tek™ II Chambered Coverglass, Thermo Fisher Scientific, Inc., USA) and placed in a special custom-designed platinum electrode applicator, as shown in Figure 20. The pure platinum (99.9%) active RF-electrode was used to minimise electrode by-products. The active electrode was immersed in the cell suspension. The effective surface of this platinum electrode was 10mm x 45mm. The amplitude modulated (AM) 13.56MHz RF source (LabEhy100, Oncotherm, Germany) was connected to the applicator via a precise impedance matching unit. The complete heating-time was 30 minutes for each. In the case of WHT 8mL of cell suspension was put in a 15mL centrifuge tube and then placed into a thermoregulated waterbath (Thermo Minder SD Mini, Taitek Corp., Japan) continuously measuring the temperature profile throughout the treatment. The complete heating-time well fit to mEHT, and was 30 min.

The heating dynamics and the treatment time at maximum temperature were the same in all treatments. After the treatments, cell suspensions were placed in 10cm diameter plastic Petri dishes (BioBik, Ina-Optika Co. Ltd., Japan) and incubated for 24h. All experiments were performed in triplicate.

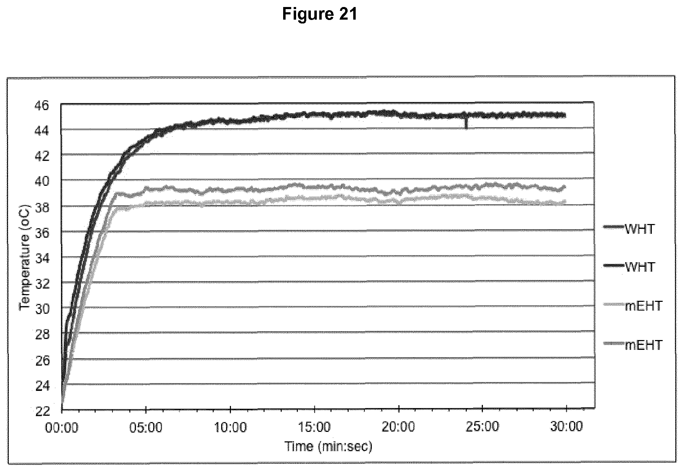

The temperature change of the cell suspension during the treatments was assessed by a four-channel fluoroptic temperature measurement system (Luxtron m3300 Biomedical Lab Kit, Lumasense Technologies, Santa Clara, CA, USA). The temperature measurement probe is an optical fibre that is 0.5 mm in diameter, which is totally insensitive to electromagnetic field. Probes 1 and 2 were used to monitor the temperature changes in the case of WHT, and probes 3 and 4 were used to measure the temperature profile of the mEHT treatment. The probes were precisely positioned on the inner surface of the slide-flask as well as at the lowest point in the centrifuge tube. The measured temperature parameters were recorded real-time (1sampling/sec) using a PC. A representative temperature measurement graph was shown in Figure 21.

The isothermal treatments were performed at 39, 40, 41, 42, 43, 44, 45 and 46°C and at 42, 43, 44, and 45°C in mEHT and WHT experiments, respectively. To identify the morphological changes of the apoptotic cells after mEHT and WHT, the cells were examined by Giemsa staining.

Cells were harvested after 3h and 24h of incubation at 37°C, washed with PBS and collected by centrifugation. Then, the cells were fixed with methanol and acetic acid (3:1) for 24h and spread on the glass slides. After drying, staining was performed with 5% Giemsa solution (pH 6.8) for 20 min, then washed with tap water. The cell samples on the slides were covered by coverslips using Eukitt (O. Kindler GmbH & Co., Germany). Cells were imaged using a conventional bright-field microscope (Olympus BX61 Olympus Corp., Japan) equipped with a standard microscope camera (Olympus DP70, Olympus Corp., Japan). Live cells were imaged using the DIC (differential interference contrast) method with an inverted microscope (Nikon Eclipse, Nikon Corp., Japan) and a conventional DSLR camera (Canon EOS D60).

To quantitatively investigate the different heat treatment-induced early apoptosis and secondary necrosis, phosphatidylserine (PS) externalisation of apoptosis was determined by analysis of propidium iodide (PI) and fluorescein isothiocyanate (FITC)-labelled Annexin V (Immunotech, Marseille, France) using Flow cytometry (Epics XL, Beckman-Coulter, Miami, FL), according to the manufacturer's instructions. Briefly, followed by the RF and WHT, cells were collected after 3h of incubation at 37°C, washed with cold PBS at 4°C and centrifuged at 1200 rpm for 3 min. The resulting pellet was mixed with the binding buffer of the Annexin V-FITC kit. FITC-labelled Annexin V (5 µl) and PI (5 µl) were added to the 490 µl suspension and mixed gently. After incubation at 4°C for 20 min in the dark, the cells were analysed by flow cytometry.

The dead cell fraction was determined by summarising the apoptotic cell fraction (Annexin V-FITC-positive fraction), the necrotic cell fraction (propidium iodide-positive cell fraction) and the secondary necrotic cell fraction (Annexin V-FITC plus propidium iodide-positive fraction).

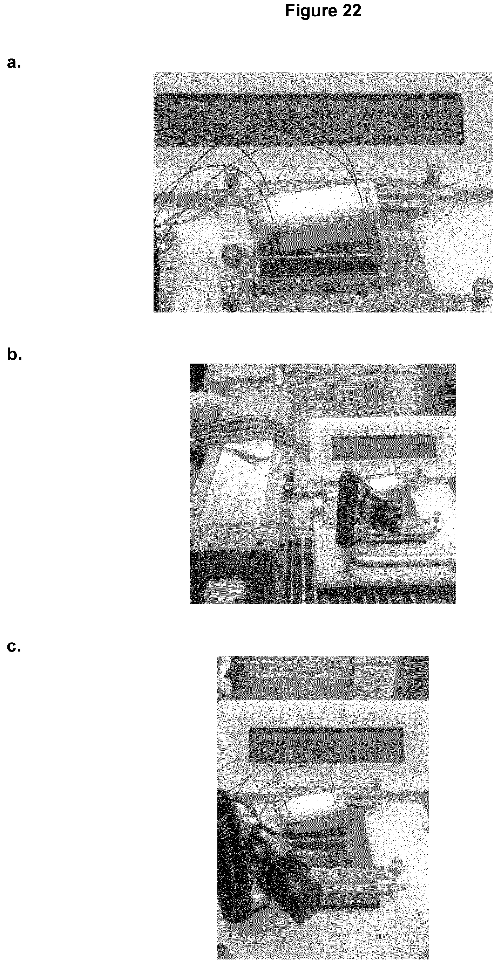

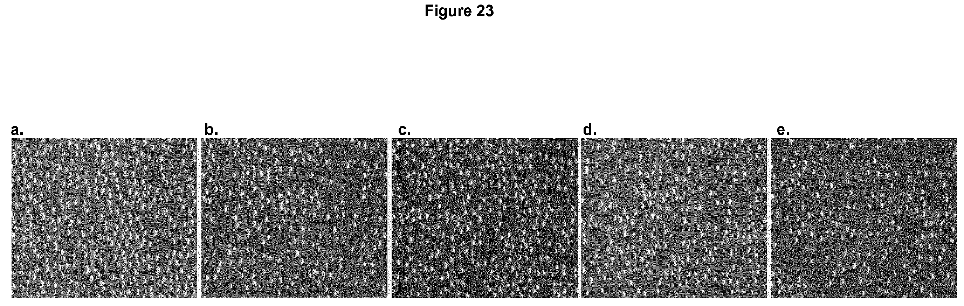

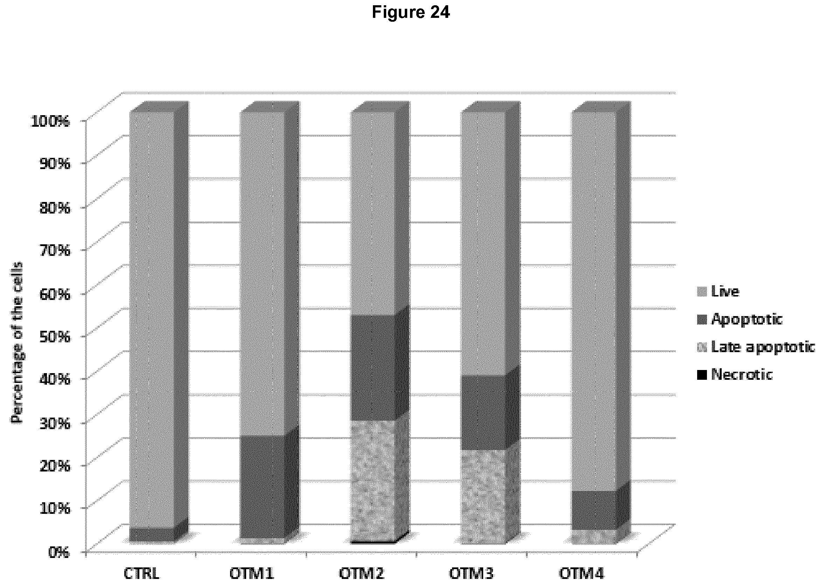

The arrangement of the experiments is shown in Figure 22. The microscopic images are shown in Figure 23 and the results measured by flow cytometry are shown in Figure 24.

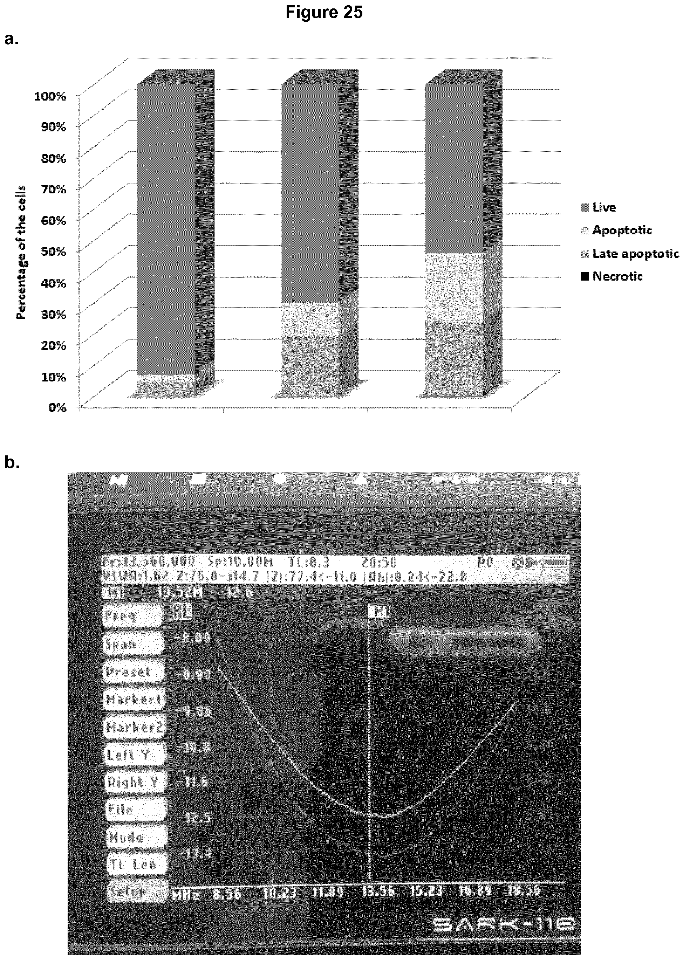

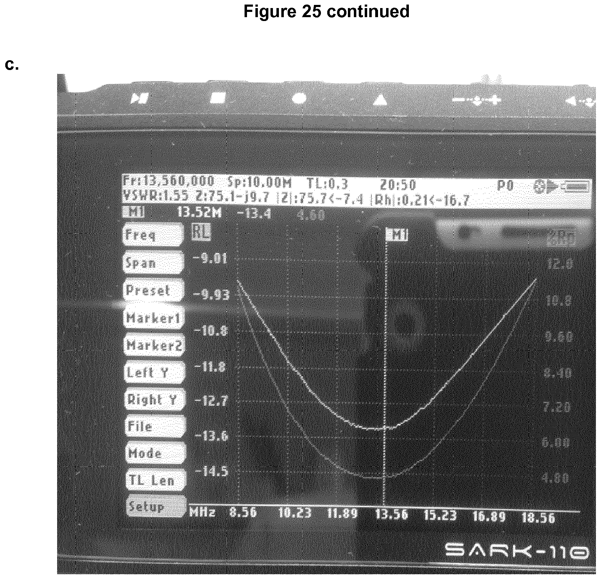

Every measurement shows higher apoptotic cell-destruction than the control, which is the natural process without treatment. The balloon matching (OTM1) has small late-apoptotic ratio compared to all others. The second impedance matching could be made by resistivity (OTM2) and by conductivity (OTM3). In this case the resistive component was more sensitive for the proper phase angle than the inductive, due to the very robust isolating property of the flask of the cell-culture. It is not so in case of *in vivo* at all. However, when the optimizing of the inductive matching is measured, the more resonant matching has larger cell-destruction (Figure 25).

## Example 2 *In vivo* experiments

There were three different setups made. Animal model was BalbC mice inoculated with C26 murine colon carcinoma cell line (C26 allograft double tumor model) subcutaneously at both of their femoral regions and were kept till the tumors reached symmetrically 10mm in diameter. C26 cells were cultivated in RPMI 1640 Glutamax medium (Invitrogen, Carlsbad, CA, USA). Inoculation was done by 7500000 cell/mL concentration liquid cell suspension, using 0,1 mL subcutaneously at each side. Incubation time for tumor growth is expected to be around two weeks. Sampling was taken 72 H posttreatment. All the points were repeated 3 times. Four setups were arranged.

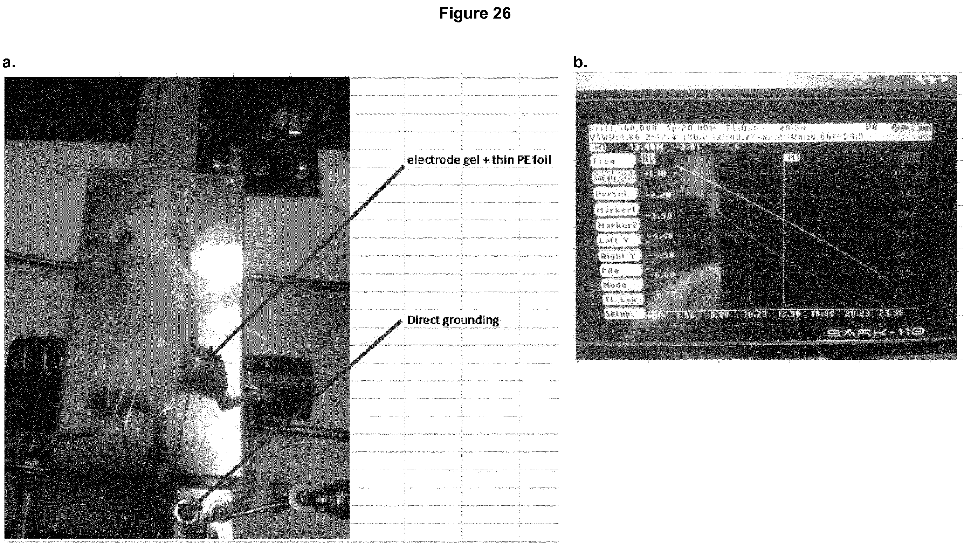

**SETUP-1.** Original electrode setup (the treating pad grounded directly to the RF ground), electrode gel (Aquasonic 100, Parker Laboratories Inc. USA) + household polyethylene (PE) foil. After the electrode was placed in the proper position onto the tumor, but before the treatment was started, a network analyzer (Sark-110) was connected to the input of the in vivo applicator to measure the impedance parameters (real and imaginary parts) and S11, Forward Reflection (input match - impedance) parameter. The setup and its matching are shown in Figure 26.

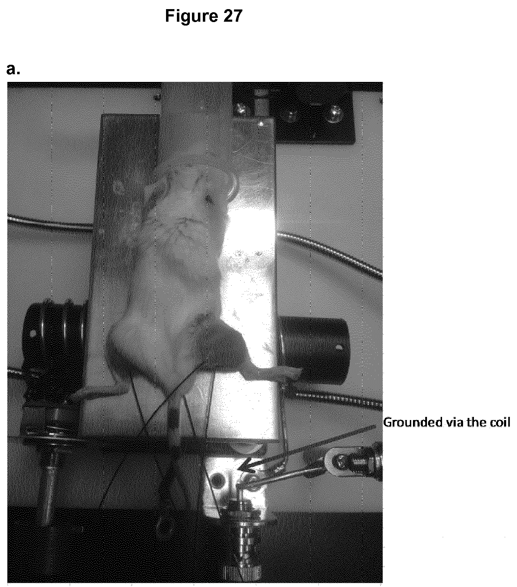

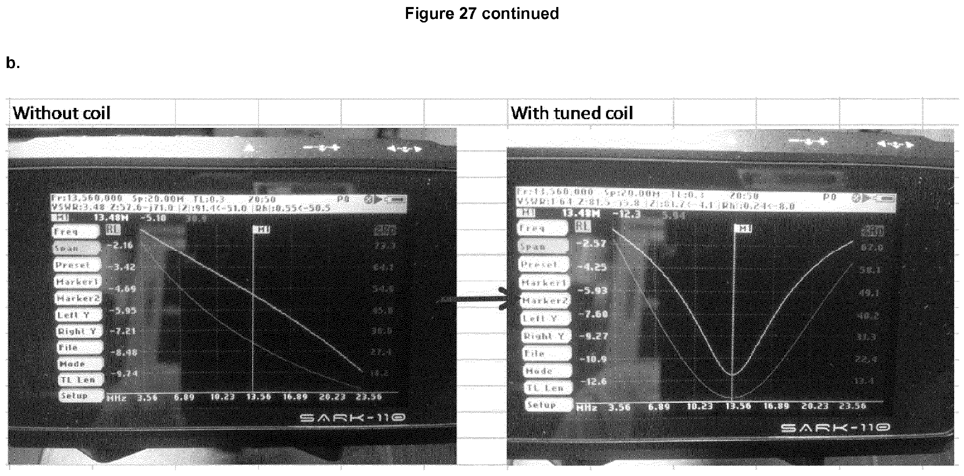

**SETUP-2.** Modified electrode grounding (the treating pad grounded via the variable inductance) applied electrode gel + PE foil measured by SHARK-110. In this setup the impedance was adjusted to form a resonant LC circuit, measured the S11 (Forward Reflection (input match - impedance)) parameter. The setup and its matching are shown in Figure 27.

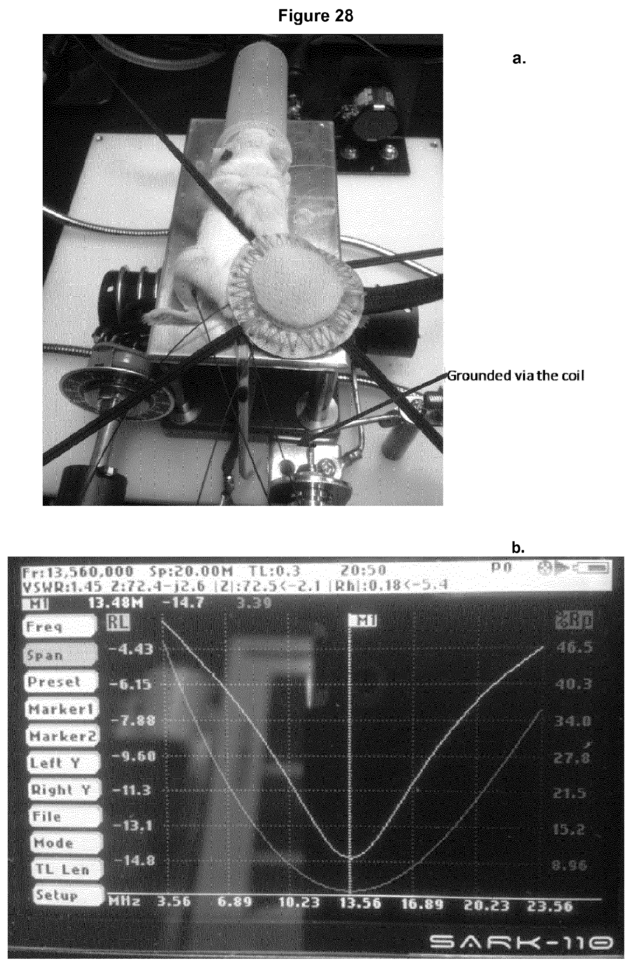

**SETUP-3.** Modified electrode grounding (the treating pad grounded via the variable inductance) without electrode gel + PE foil, the textile electrode was placed directly to the skin surface. In this setup the impedance was adjusted to form a resonant LC circuit. The setup and its matching are shown in Figure 28.

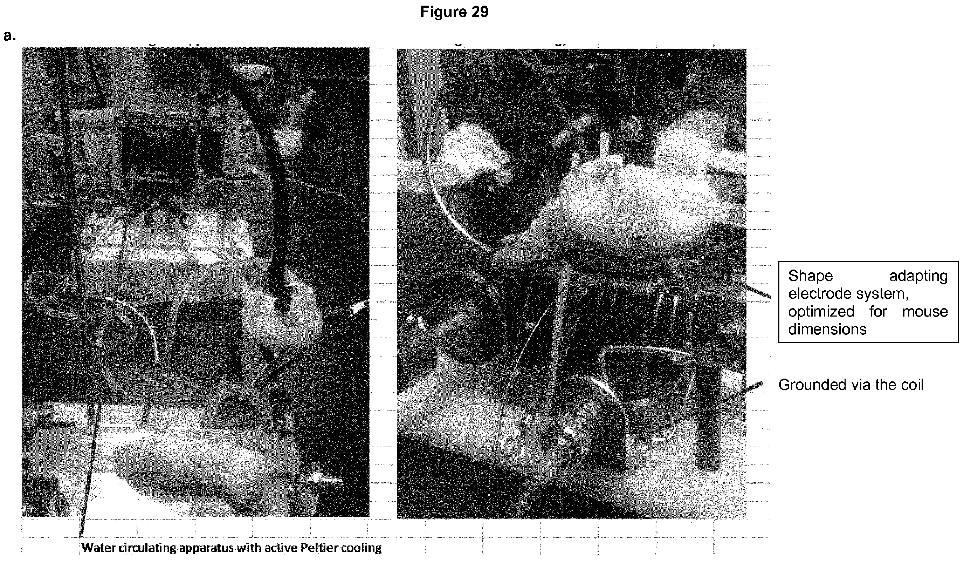

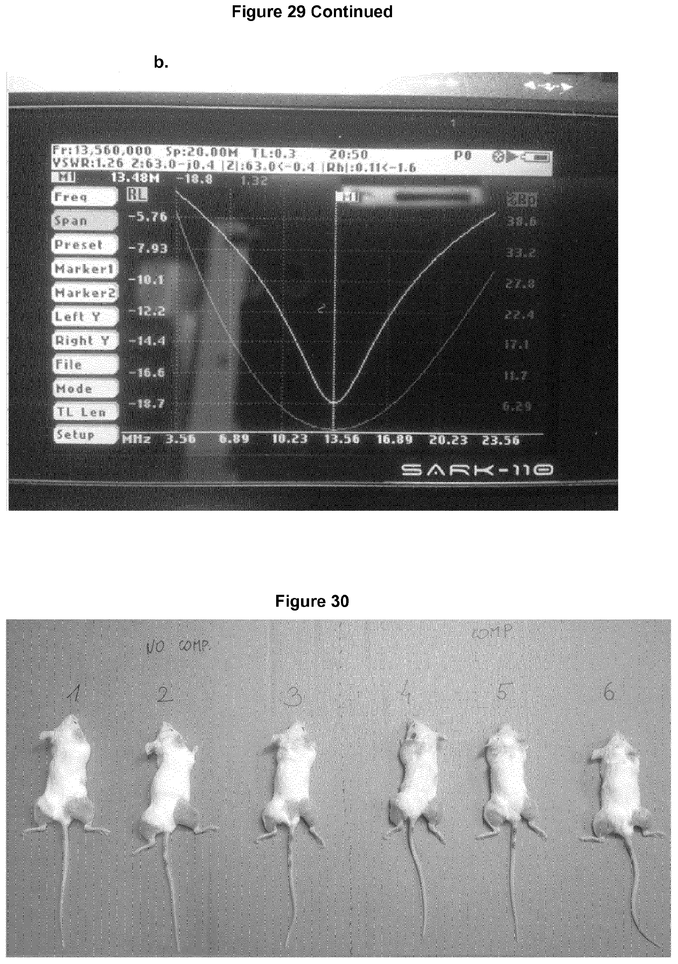

**SETUP-4.** Modified electrode grounding (the treating pad grounded via the variable inductance) without electrode gel + PE foil, the textile electrode was placed directly to the skin surface. In this setup the impedance was adjusted to form a resonant LC circuit. Intensive active cooling by sponge moisture with water and placed on the top of the electrode, was applied on the tumor surface enabling to deliver higher dose RF energy (instead the average 2kJ we applied 2.5kJ in these cases). The setup and its matching are shown in Figure 29.

Figure 30 shows the mice (from SETUP-1 and SETUP-2) immediately after their sacrifice.

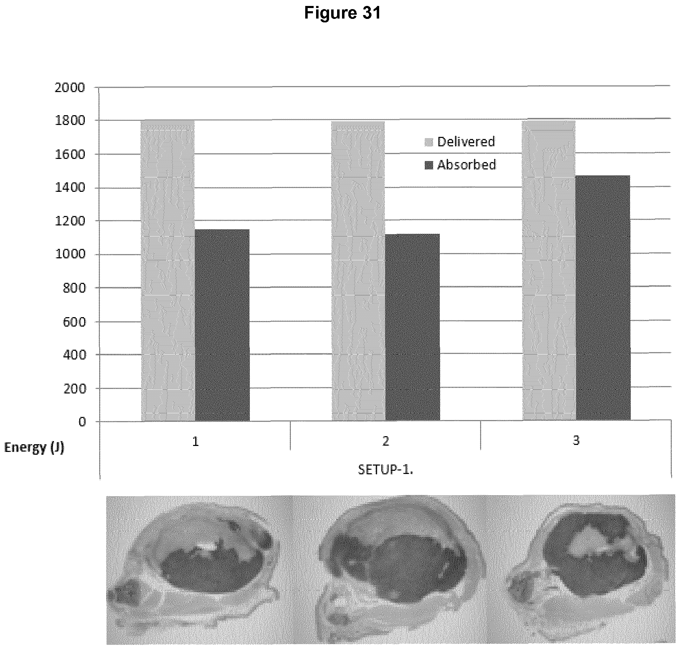

The results show well the importance of the in-situ matching at the treated target. The results of SETUP-1 shows definitely less absorption energy than delivered, (measured by the power integrated in time applied) and the cell-destruction (measured on morphologic pictures, with simple comparison of the relative destructed areas), is located mainly on the surface of the tumor, near the electrode. When the absorption is a little bit more, (shown by columns on the diagrams) than the cell-destruction is deeper (shown by morphological pictures below the diagrams). The morphological evaluation was made on the complete cross section, which was stained by hematoxylin-eosin (HE) standard conventional method (Figure 31).

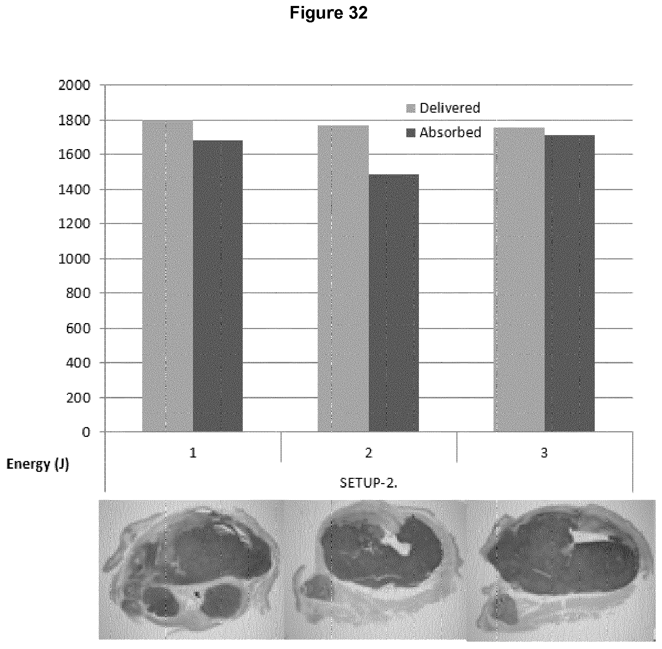

The well-matched situation by induction in SETUP-2 shows higher absorbed energy, but the cell-destruction is less than was in SETUP1. This is the typical case, when the phase angle of the tumor is far not the same as the phase angle of the resultant impedance (Figure 32).

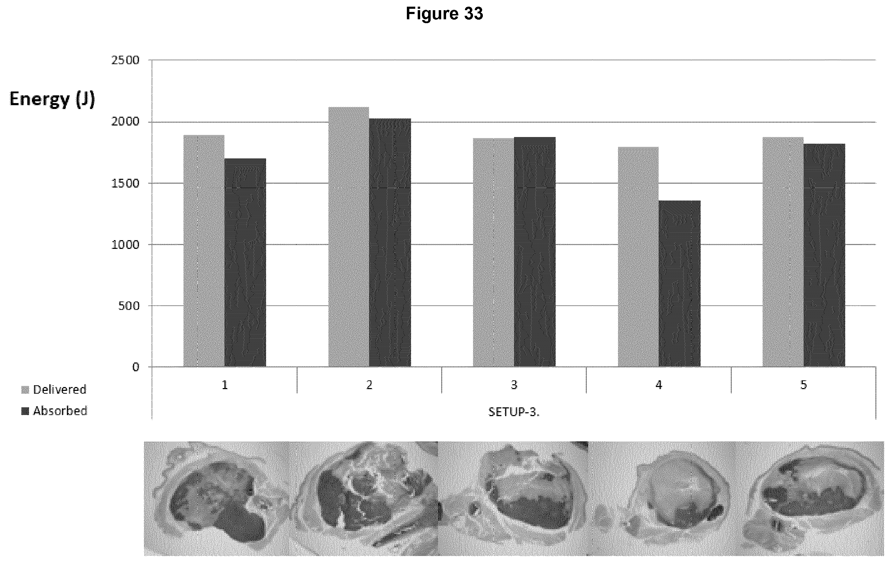

When the impedance is fitted by lowering the resistivity and increasing the capacitance (SETUP-3), we find the optimal cell-destruction (the best destruction compared to other SETUPs), because the tumor phase angle became near to zero. The ratio of the delivered and absorbed energy here has no central role, the important factor is the proper matching for the tumor (Figure 33).

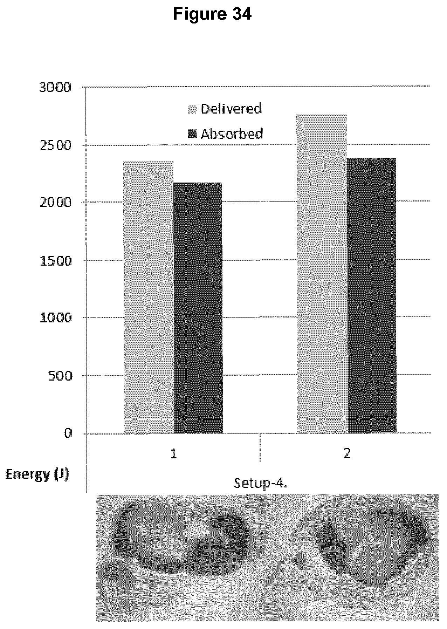

The resistivity and the capacity could be modified by shape adapting electrode (Patent pending), which gives slightly better results (SETUP-4) than the previous setup. The modification of the resistivity is made simple by the pressure of the electrode, by the conductive gel-layer and by the size of the touching area, while the capacity is modified also by pressure and the isolation layer (PE or hygienic paper or others). This result shows again, that the ratio of the delivered and absorbed energy has no primary influence, because when we have the best ratios (#3, and #5) the results were worse than in the case of bad rations (like #1, and #4). (Figure 34).

**Claims**

1. A radiofrequency hyperthermia device comprising:

a radiofrequency carrier signal generator (1) configured and adapted to generate a radiofrequency carrier signal (S1) having a carrier frequency;
a radiofrequency amplifier (4) configured and adapted to amplify the radiofrequency carrier signal;
an electrode (6) and a counter-electrode (7), wherein a RF current is generated between the electrode (6) and the counter-electrode (7) for capacitive coupled energy transfer;
a first impedance measuring means (2) configured and adapted to measure a first impedance (I1) caused by the device;
a first impedance matching means (3) configured and adapted to match the first impedance (I1) of the radiofrequency carrier signal (S1) measured by the first impedance measuring means (2) to a predetermined value;
a second impedance measuring means (5) configured and adapted to measure a second impedance (I2) ; and
at least one second impedance matching means (8 or 8');
**characterized in that** the at least one second impedance matching means (8 or 8') is configured and adapted to match the second impedance (I2) measured by the second impedance measuring means (5) to the predetermined value.

2. The radiofrequency hyperthermia device according to claim 1, wherein the at least one second impedance matching means (8 or 8') is selected from the group consisting of: a tuneable coil, a capacitor and a coil in serial connection.

3. The radiofrequency hyperthermia device according to claim 1 or 2, wherein the at least one second impedance matching means (8 or 8') is mounted on the electrode (6) and/or the counter-electrode (7).

4. The radiofrequency hyperthermia device according to any one of claims 1 to 3, wherein the at least one second impedance matching means (8 or 8') is integrated into the electrode (6) and/or the counter-electrode (7).

5. The radiofrequency hyperthermia device according to any one of claims 1 to 4, wherein the electrode (6) or the counter-electrode (7) is adaptable to any shape of a target (17).

6. The radiofrequency hyperthermia device according to claim 5, wherein the target (17) is a tissue, an organ, a part of or the whole of a human or an animal body.

7. The radiofrequency hyperthermia device according to any one of claim 1 to 6, wherein the first impedance measuring means (2) and the first impedance matching means (3) are integrated into the radiofrequency carrier signal generator (1).

8. The radiofrequency hyperthermia device according to any one of claims 1 to 7, further comprising:

a modulation signal generator (9) configured and adapted to generate a modulation signal (SM);

a modulator **(11)** configured and adapted to convert the radiofrequency carrier signal **(S2)** after the first impedance matching and the modulation signal **(SM)** generated by the modulation signal generator **(9)** to a modulated radiofrequency carrier signal **(S2M)**;

wherein the radiofrequency amplifier **(4)** is configured and adapted to amplify the modulated carrier signal **(S2M)** converted by the modulator **(11).**

9. The radiofrequency hyperthermia device according to claim 8, wherein the modulator is configured and adapted to modulate an amplitude of the radiofrequency carrier signal based on the modulation signal.

10. The radiofrequency hyperthermia device according to claim 8 or 9, wherein the modulator is configured and adapted to further modulate a frequency of the radiofrequency carrier signal.

11. The radiofrequency hyperthermia device according to any one of claims 8 to 10, further comprising:

a sensor **(12)** configured and adapted to detect a reflected or transmitted feedback signal **(ST)**; wherein

the modulation signal generator **(9)** receives a feedback signal **(ST)** detected by means of the sensor **(12)** for further generating the modified modulation signal **(SM')** compensated by the feedback signal; and

the modulator **(11)** is configured and adapted to convert the radiofrequency carrier signal **(S2)** obtained after the first impedance matching and a modified modulation signal **(SM')** compensated by a feedback signal **(ST)** to a target modified modulated radiofrequency carrier signal **(S2M')**.

12. A method for matching an impedance of a radiofrequency carrier signal of the device of claim 1 comprising the septs of:

(a) providing a radiofrequency carrier signal **(S1)** by means of a radiofrequency carrier generator **(1)**;

(b) measuring an impedance **(I1)** of the radiofrequency carrier signal **(S1)** by means of a first impedance measuring means **(2)**;

(c) matching the impedance **(I1)** of the radiofrequency carrier signal **(S1)** by means of a first impedance matching means **(3)** to obtain an radiofrequency carrier signal **(S2)** obtained after the first impedance matching;

(d) amplifying the radiofrequency carrier signal **(S2)** obtained after the first impedance matching by means of a radiofrequency amplifier **(4)** to obtain an amplified radiofrequency carrier signal **(S3)**;

(e) measuring an impedance **(I2)** directly at a target **(17)** by means of a second impedance measuring means (5);

(f) compensating the impedance **(I2)** by means of at least one second impedance matching means **(8 or 8')**;

(g) directing the impedance matched carrier signal to an electrode **(6)**; and

(h) generating a RF current between the electrode **(6)** and a counter-electrode **(7)** for capacitive coupled energy transfer.

13. The method according to Claim 12, wherein the step (d) is replaced with the following steps (d1 a) and (d2a):

(d1a) modulating the radiofrequency carrier signal **(S2)** with a modulation signal **(SM)** provided by means of a modulating signal generator **(9)** to obtain a modulated radiofrequency carrier signal **(S2M)**;

(d2a) amplifying the modulated radiofrequency carrier signal **(S2M)** by means of a radiofrequency amplifier **(4)** to obtain a modulated radiofrequency carrier signal **(S3M)** after amplification.

14. The method according to Claim 12, further comprising the steps (i) and (j):

(i) detecting a feedback signal **(ST)** received by means of a sensor **(12)**;

(j) sending the feedback signal **(ST)** received to the modulation signal generator for providing a modified modulation signal **(SM')** compensated by the feedback signal instead of the modulation signal in the step (d1);

wherein the step (d) is replaced with the following steps (d1 b) and (d2b):

(d1b) modulating the radiofrequency carrier signal **(S2)** with a modified modulation signal **(SM')** provided by means of a modulating signal generator **(9)** to obtain a target modified modulated radiofrequency carrier signal **(S2M')**;

(d2b) amplifying the target modified modulated radiofrequency carrier signal **(S2M')** by means of a radiofrequency amplifier **(4)** to obtain a target modified modulated radiofrequency carrier signal **(S3M')** after amplification.

# Figures

## Figure 1

a.

b.

## Figure 2

$$Z_1=R_1+jX_1 \quad Z_2=R_2+jX_2$$

$$Z=R_1+R_2+j(X_1+X_2)$$

## Figure 3

$$Z_1=R_1+jX \quad Z_2=R_2-jX$$

$$Z=R_1+R_2$$

# Figure 4

a

Larger tumor

Real part of impedance (resistance)

$R_t$

$R_h$

$\phi_h$

$\phi_t$

$Z_h=R_h-jX_h$

$-jX_h$

$Z_t=R_t-jX_t$

$-jX_t$

healthy-connective

tumor

b

Smaller or dispersed tumor

Real part of impedance (resistance)

$\phi_h$ $R_t$ $R_h$

$\phi_t$

$-jX_h$

Imaginary part of impedance (reactance)

$Z_h=R_h-jX_h$

healthy-connective

$-jX_t$

$Z_t=R_t-jX_t$

dispersed tumor

## Figure 5

# Figure 6

**a.**

**b.**

# Figure 7

a.

b.

**Figure 8**

a.

b.

## Figure 9

The treated object is a part of the electric circuit

## Figure 10

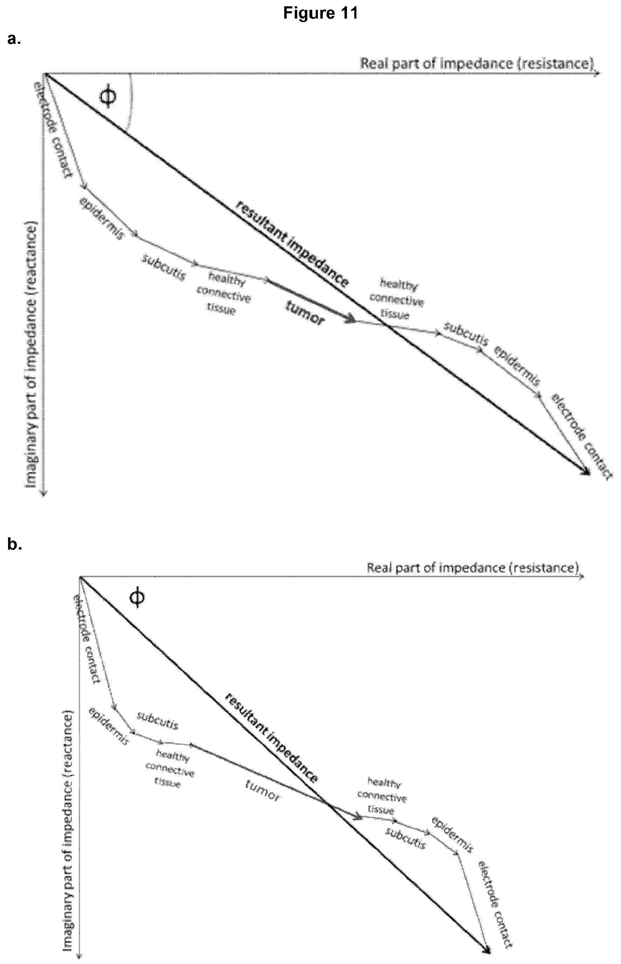

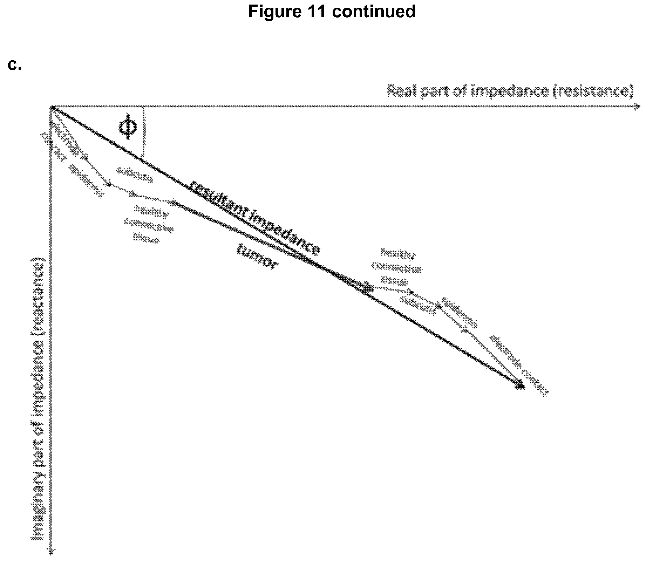

# Figure 11

a.

b.

**Figure 11 continued**

c.

# Figure 12

**a.**

**b.**

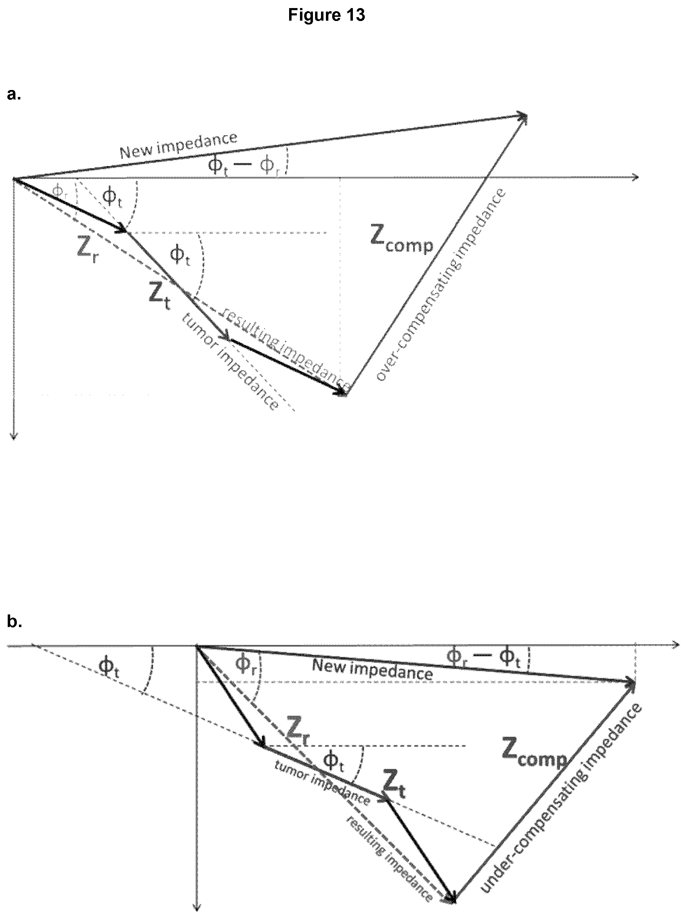

## Figure 13

**a.**

**b.**

## Figure 14

a.

b.

# Figure 15

a.

b.

## Figure 16

a.

b.

c.

d.

## Figure 17

a.

b.

## Figure 18

a.

b.

## Figure 19

a.

b.

# Figure 19 Continued

c.

main circuit

1 3 I1 2 4 5 x(t) 6 17 7 8 8′

S1 S2 S3 I2 S4 S ST

18

d.

main circuit

1 F(t) 2 3 11 4 x(t) 5 6 17 7 8 8′

S1 I1 S2 S2M S3M I2 S4M ST

18

SM f(x(t))sin(ωt)

modulation circuit

18 sin(ωt)

9 10

# Figure 19 Continued

**e.**

**f.**

# Figure 19 Continued

**g.**

**h.**

# Figure 19 Continued

i.

## Figure 20

a.

LabEHY-100 RF generator (13.56MHz)

Platinum plate as an active electrode immersed into the cell suspension

Platinum electrode plastic holder

LabTek coverglass chamber

Impedance matching unit

Copper plate as a counter-electrode

Cell suspension in the medium

b.

Fluoro-optical temperature sensor

Temperature feedback

Heat controller

Temperature measurement

Water-bath

**Figure 21**

# Figure 22

a.

b.

c.

Figure 23

Figure 24

# Figure 25

a.

b.

**Figure 25 continued**

c.

Figure 26

a.

electrode gel + thin PE foil

Direct grounding

b.

EP 3 231 478 A1

**Figure 27**

a.

Grounded via the coil

Figure 27 continued

b.

Without coil

With tuned coil

# Figure 28

a.

Grounded via the coil

b.

**Figure 29**

Shape adapting electrode system, optimized for mouse dimensions

Grounded via the coil

a.

Water circulating apparatus with active Peltier cooling

**Figure 29 Continued**

b.

**Figure 30**

# Figure 31

**Figure 32**

Figure 33

# Figure 34

Setup-4.

Europäisches
Patentamt

European
Patent Office

Office européen
des brevets

## EUROPEAN SEARCH REPORT

Application Number

EP 16 16 5109

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/251234 A1 (KANZIUS JOHN [US] ET AL) 10 November 2005 (2005-11-10) * abstract; claim *; figure * * * paragraphs [0026] - [0102] * ----- | 1-14 | INV. A61N1/40 A61N5/10 A61K41/00 B82Y5/00 |
| A | EP 2 174 689 A1 (ONCOTHERM KFT [HU]) 14 April 2010 (2010-04-14) * the whole document * ----- | 1-14 | |
| A | US 2016/074668 A1 (NUNEZ ALBERT [US] ET AL) 17 March 2016 (2016-03-17) * the whole document * ----- | 1-14 | |
| A | US 4 674 481 A (BODDIE JR ARTHUR W [US] ET AL) 23 June 1987 (1987-06-23) * the whole document * ----- | 1-14 | |

TECHNICAL FIELDS
SEARCHED      (IPC)

A61N
A61K
B82Y

The present search report has been drawn up for all claims

| Place of search | Date of completion of the search | Examiner |
|---|---|---|
| Munich | 19 September 2016 | Scheffler, Arnaud |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 16 16 5109

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2016

| Patent document cited in search report | | Publication date | Patent family member(s) | | Publication date |
|---|---|---|---|---|---|
| US 2005251234 | A1 | 10-11-2005 | CA | 2562625 A1 | 24-11-2005 |
| | | | EP | 1758648 A1 | 07-03-2007 |
| | | | EP | 2345453 A2 | 20-07-2011 |
| | | | JP | 4579975 B2 | 10-11-2010 |
| | | | JP | 2007536016 A | 13-12-2007 |
| | | | JP | 2010167283 A | 05-08-2010 |
| | | | US | 2005251234 A1 | 10-11-2005 |
| | | | US | 2005273143 A1 | 08-12-2005 |
| | | | WO | 2005110261 A2 | 24-11-2005 |
| | | | WO | 2005118065 A2 | 15-12-2005 |
| | | | WO | 2005120639 A2 | 22-12-2005 |
| EP 2174689 | A1 | 14-04-2010 | CN | 102176948 A | 07-09-2011 |
| | | | EP | 2174689 A1 | 14-04-2010 |
| | | | EP | 2349474 A1 | 03-08-2011 |
| | | | RU | 2011119020 A | 20-11-2012 |
| | | | US | 2012065714 A1 | 15-03-2012 |
| | | | WO | 2010043372 A1 | 22-04-2010 |
| US 2016074668 | A1 | 17-03-2016 | GB | 2531619 A | 27-04-2016 |
| | | | US | 2016074668 A1 | 17-03-2016 |
| | | | WO | 2016040867 A1 | 17-03-2016 |
| US 4674481 | A | 23-06-1987 | NONE | | |

EPO FORM P0459

For more details about this annex : see Official Journal of the European Patent Office, No. 12/82